# EUROPEAN PATENT SPECIFICATION

(11) **EP 1 253 864 B1**
(45) Date of publication and mention of the grant of the patent: **24.10.2012**
(21) Application number: 00951812.7
(22) Date of filing: 03.08.2000
(51) Int. Cl.: A61B 17/70, A61B 17/74, A61B 17/86, A61F 2/36

(54) **EXPANDING BONE IMPLANTS**
AUFWEITBARE KNOCHENIMPLANTATE
IMPLANTS OSSEUX EXPANSIBLES

(30) Priority: 27.01.2000 WO PCT/IL00/00055; 27.01.2000 WO PCT/IL00/00058; 26.03.2000 IL 13527300
(43) Date of publication of application: 06.11.2002
(73) Proprietor: Kyphon SÀRL, 2000 Neuchâtel (CH)
(72) Inventor: SHAVIT, Ronen, 69413 Tel-Aviv (IL); MAGAL, Elad, 47220 Ramat-Hasharon (IL); LAMAROVITZ, Ilan, 64511 Tel-Aviv (IL); SHARON, Simon, 38900 Caesaria (IL)
(74) Representative: Viering, Jentschura & Partner
(86) International application number: PCT/IL2000/000471
(87) International publication number: WO 2001/054598

(56) References cited:
- WO-A-96/32899
- WO-A-98/38918
- DE-A- 1 810 799
- DE-A- 19 612 276
- DE-U- 8 716 073
- US-A- 4 274 163
- US-A- 4 346 708
- US-A- 4 632 101
- US-A- 5 376 123
- US-A- 5 578 035
- US-A- 5 658 310
- US-A- 5 827 289
- DATABASE WPI Section PQ, Week 199708 Derwent Publications Ltd., London, GB; Class P31, AN 1997-081321 XP002154628 -& JP 08 322848 A (NARUSHIMA M), 10 December 1996 (1996-12-10)
- S.L.WEISSMAN AND R.SALAMA: "Trochanteric Fractures of the Femur" CLINICAL ORTHOPAEDICS, no. 67, 1969, pages 143-150, XP000964714 cited in the application

## Description

### FIELD OF THE INVENTION

The present invention relates to medical implants with expanding portions.

### BACKGROUND OF THE INVENTION

US-A-5 578 035 discloses an expandable bone marrow cavity fixation device comprising a tubular body having a hollow interior, said tubular body including an anchoring portion at a tip of the tubular body, the anchoring portion being provided peripherally with a plurality of through holes communicating said hollow interior with the outside of the tubular body and provided with a plurality of elements received movably in the through holes so as to be movably radially outwardly, wherein the length of the anchoring portion is less than 60% of the length of the tubular body.

WO-A-9838918 discloses an implant having a shaft defining an inflation lumen therethrough, and an inflatable anchor portion in form of a flexible membrane at a tip of the shaft and connected to said lumen, wherein said anchor portion includes a plurality of protrusions extending from the membrane in a transversal manner.

Pedicle screws are screws that are driven into a vertebra. One exemplary use of pedicle screws is as an anchor for spinal stabilization and/or fusion plates. Another exemplary use is as an anchor for spinal support and/or bending bars, for example for treating spondylolisthesis.

In a standard pedicle screw fixation procedure, a hole is drilled in the cortex and in the cancellus portion of a vertebra body and pedicle, the diameter of the hole being substantially the same as a diameter of a shaft of the pedicle screw being used. Then, the screw is screwed into the hole, with the threading of the screw engaging both the cortex and the cancellus portions of the vertebra.

Many types of hip implants are known in the art. A common problem with hip implants and with repairing fractures near the femoral head (the proximal femur) is that the bone may crumble under the strain applied by the various artificial implants used. This is especially true in patients where the fracture has rotational instability and/or in patients with weak bones, for example due to osteoporosis.

To prevent breakage of femoral neck nails in a nail-plate combination implant, Weissman and Salma, in "Trochanteric Fractures of the Femur", in Clinical Orthopaedics no. 67, 1969, the disclosure of which is incorporated herein by reference, suggested providing a strut between the middle of the nail and the plate, to distribute the load.

PCT publication WO 98/38918, from which this application claims priority for the US, describes inflatable axial intra-medullar nails for bones.

### SUMMARY OF THE INVENTION

An aspect of some embodiments of the invention relates to implants that anchor in cancellus bone by using an expansion of a part of the implant to displace cancellus bone. Optionally, part of the implant expands against or inside a cortical portion of the bone as well.

The invention provides an implant according to claim 1. Further embodiments of the invention are described in the dependent claims.

One potential advantage of inflation is that the volume of the implant is definitely increased. Another potential advantage of inflation is that the implant can remain in position and does not shorten during inflation, thus allowing the implant to be positioned prior to expansion, with less of a danger that the implant will move in an undesirable manner during the expansion. In some implementations, the implant may shorten slightly during expansion, thereby increasing an axial compression caused by the implant. A pattern of protrusions on the expanding part may prevent or direct such contraction behavior. In some implementations, some axial extension rather than contraction, may be provided by the expansion.

Exemplary uses of such implants include bone anchors (for fixing an implant, a rod or an external plate to a bone), supports for artificial joints and attaching bone pieces to each other.

An aspect of some exemplary embodiments of the invention relates to a pedicle screw having one or more expandable portions. In an exemplary embodiment of the invention, an intra-cancellus portion of the screw expands, maintaining the screw in place (axially and/or rotationally) by anchoring inside a cancellus portion of the vertebra body. Alternatively or additionally, a portion of the screw near the cortex expands, preventing retraction through the hole in the cortex. Alternatively or additionally, the cortex is held between the expanding portion and a fixed shaft thickening of the screw outside the cortex. Alternatively or additionally, the expansion is of a screw portion in the hole in the cortex, engaging the cortex by friction. Alternatively or additionally, the expansion is in a screw portion outside the vertebra, possibly cooperating with an expansion portion inside the vertebra.

An aspect of some exemplary embodiments of the invention relates to providing an expandable insert for a medulla portion of a femoral shaft, which insert operates as an anchor for a different implant, such as an artificial hip joint or a femoral neck nail. The anchor may be integral with the different implant or be a separate implant that is loosely or tightly coupled to the anchor portion. In an exemplary embodiment of the invention, the anchor comprises an expanding metal membrane, such as a balloon with a plurality of protrusions defied thereon, to engage an inner surface of the cortex of a femur into which the anchor is inserted. Prior to inflation, the membrane and/or the bars may be folded, to have a smaller diameter.

An aspect of some exemplary embodiments of the invention relates to proximal femoral nails with expanding portions, to assist in the engaging of the bone by the nail. In an exemplary embodiment of the invention, the expanding portion is near a tip of the nail, which may be, for example, inserted through a femoral neck and into a femur head. The base of the nail may be for example, fixed into the femur or allowed at least some axial motion (dynamic compression).

In an exemplary embodiment of the invention, a proximal femoral nail is inserted through an eye of an expandable anchor inserted into the medullar channel of the femoral shaft. Thus, at least some of the strain from the nail is passed directly into the implant and distributed along the femur. It should be noted that such femoral neck nails may also be used for treating other fractures of the proximal femur, for example fractures in the pre-trocantric region of the femur. In addition, such nails may be used to treat other fractures of bone ends, such as cleavage fractures (e.g., separation of condyles).

An aspect relates to guiding an expandable implant over a guide wire (flexible) or a guiding rod (stiff). In an exemplary embodiment of the invention, the implant, for example, a nail, includes a lumen for the guide wire. Alternatively or additionally, the nail includes a slot along its axis for the wire. In an exemplary embodiment of the invention, at least a part of the implant is inflatable. Alternatively, the implant uses another expansion mechanism.

In some embodiments of the invention, the implant is simply advanced over the wire. Alternatively, the implant is screwed in, while the wire is in place, for example using an open wrench, having a channel in the screwdriver for the guide or the guide passing to one side of the screwdriver (or other advancing tool). Optionally, a guide bore for the implant is pre-drilled only in the cortex portion of the bone. A narrower bore, or no bore at all, is drilled in the cancellus bone portion of the bone.

There is thus provided in accordance with an exemplary embodiment of the invention, an implant adapted to anchor in cancellus bone, in non-axial orientation in a bone.

Optionally, said non-axial orientation comprises an angle of at least 30° to the axis. Optionally, said non-axial orientation comprises an angle of at least 50° to the axis. Optionally, said non-axial orientation comprises an angle of at least 70° to the axis.

Optionally, the anchor portion comprises less than 50% of a length of said implant. Optionally, the anchor portion comprises less than 30% of a length of said implant.

In an exemplary embodiment of the invention, the implant has a length less than 30% of a length of a maximal extent of a bone for which said implant is designed. Optionally, the implant has a length less than 20% of a length of a maximal extent of a bone for which said implant is designed. Optionally, the implant has a length less than 10% of a length of a maximal extent of a bone for which said implant is designed.

In accordance with the invention, said anchor portion is at a distal tip of said shaft. Optionally, said implant comprises a distal end adapted for insertion through cancellus bone.

In accordance with the invention, said anchor portion is at a proximal tip of said shaft.

In accordance with the invention, said anchor portion is at a center portion of said shaft. Optionally, said shaft has at least one end adapted to attach an object thereto. Alternatively, said shaft has two ends each adapted to attach an object thereto.

In an exemplary embodiment of the invention, said implant comprises a base portion having a greater diameter than said shaft and adapted to remain outside said bone. Optionally, said base is adapted to urge an object against an outside of said bone. Optionally, said object comprises a bone plate.

In an exemplary embodiment of the invention, said base is adapted for attachment of an object thereto. Optionally, said object comprises a rod. Optionally, said rod has a thin surface texture. Alternatively or additionally, said surface texture comprises a plurality of radial notches.

In an exemplary embodiment of the invention, said implant comprises a base portion having a diameter that is not greater than said shaft and adapted to remain inside said bone.

In an exemplary embodiment of the invention, said implant comprises a separate axial over tube adapted to be fixed to said bone and sized to enclose said shaft. Optionally, said shaft is free to move axially in said tube. Alternatively, said shaft is elastically axially coupled to said tube. Alternatively, said shaft is rigidly coupled to said tube.

In an exemplary embodiment of the invention, said implant comprises a second shaft having at least a second inflatable anchor portion defined thereon and comprising a coupling between said shaft and said second shaft. Optionally, said second shaft is adapted for axial inserting in said bone. Alternatively or additionally, said coupling comprises a through hole in said second shaft. Alternatively, said coupling comprises a through hole in said shaft. Alternatively, said coupling comprises a notch in said second shaft.

In an exemplary embodiment of the invention, said implant comprises a strut adapted to couple to said first and second shafts.

In an exemplary embodiment of the invention, said implant comprises a strut adapted to at least contact said shaft. Optionally, said strut is coupled to said shaft.

In an exemplary embodiment of the invention, said inflatable anchor portions is adapted to expand wholly in cancellus bone and not contact cortical bone.

In an exemplary embodiment of the invention, said implant comprises an expanding cortical anchoring portion, adapted to expand in contact with cortical bone.

In an exemplary embodiment of the invention, said implant comprises a second inflatable cancellus anchoring portion, adapted to engage a cancellus bone portion of another section of said bone, separated by a fracture line from a section which anchors said anchoring portion.

In an exemplary embodiment of the invention, said shaft is not inflatable.

In an alternative exemplary embodiment of the invention, said shaft inflates to a lesser degree than said anchoring portion.

In an exemplary embodiment of the invention, at least part of said shaft is externally threaded. Optionally, said threading is adapted to engage cortical bone. Alternatively or additionally, said threading is adapted to engage cancellus bone. Alternatively or additionally, at least part of said anchoring portion is externally threaded. Optionally, said anchoring portion threading is damaged by said inflation. Alternatively, said anchoring portion threading is not damaged by said inflation.

In an exemplary embodiment of the invention, said implant comprises matched threading on said anchoring portion and said shaft.

Alternatively or additionally, said implant comprises a source of pressurized fluid for inflating said inflatable portion. Optionally, said fluid comprises a saline solution. Alternatively, said fluid hardens after said inflation.

In an exemplary embodiment of the invention, said inflation elastically deforms said inflatable portion. Alternatively or additionally, said inflation plastically deforms said inflatable portion.

In an exemplary embodiment of the invention, said shaft and said inflatable portion are comprised of a metal. Optionally, said metal is stainless steel. Alternatively or additionally, said metal comprises titanium.

In an exemplary embodiment of the invention, said shaft and said inflatable portion are comprised of a same material.

In an exemplary embodiment of the invention, at least a part of said inflatable portion is metallurgically treated to have better elongation properties than said shaft.

In an exemplary embodiment of the invention, said shaft and said inflatable portion are comprised of different materials.

In an exemplary embodiment of the invention, said shaft defines a guide channel for a guiding element. Optionally, said guiding element is a guide wire. Alternatively, said guiding element is a rigid element. In an exemplary embodiment of the invention, said guide channel comprises a lumen. Alternatively, said guide channel comprises a slot along an exterior part of said shaft.

In an exemplary embodiment of the invention, said inflatable portion comprises a thin membrane. Optionally, said inflatable portion comprises a plurality of protrusions on said membrane. Optionally, said plurality of protrusions comprises at least one axially aligned bar. Alternatively or additionally, said plurality of protrusions comprises at least one trans-axially aligned bar. Alternatively or additionally, said plurality of protrusions comprises a spirally arranged bar. Alternatively or additionally, said plurality of protrusions comprises a mesh pattern. Alternatively or additionally, said plurality of protrusions comprises an array arrangement of protrusions. Alternatively or additionally, said membrane has a smooth surface texture. Alternatively or additionally, said membrane has a rough surface texture.

In an exemplary embodiment of the invention, said protrusions resist bending of said inflatable portion. Alternatively or additionally, said protrusions resist bending of said inflatable portion relative to said shaft.

In an exemplary embodiment of the invention, said inflatable portion and said shaft have substantially a same diameter prior to inflation. Alternatively, said inflatable portion has a substantially smaller diameter than said shaft prior to inflation. Alternatively, said inflatable portion has a substantially greater diameter than said shaft prior to inflation.

In an exemplary embodiment of the invention, said implant has a length of less than 150 mm. Optionally, said implant has a length of less than 100 mm. Optionally, said implant has a length of less than 75 mm. Optionally, said implant has a length of less than 50 mm. Optionally, said implant has a length of less than 30 mm.

In an exemplary embodiment of the invention, said implant has a shaft diameter of less than 15 mm. Optionally, said implant has a shaft diameter of length of less than 10 mm. Optionally, has a shaft diameter of length of less than 5 mm. Optionally, said implant has a shaft diameter of length of less than 3 mm.

In an exemplary embodiment of the invention, said implant has an expansion ratio of at least 1:1.2 of its diameter before and after inflation. Optionally, said implant has an expansion ratio of at least 1:1.4 of its diameter before and after inflation. Optionally, said implant has an expansion ratio of at least 1:1.7 of its diameter before and after inflation. Optionally, said implant has an expansion ratio of at least 1:2.2 of its diameter before and after inflation. Optionally, said implant has an expansion ratio of at least 1:3 of its diameter before and after inflation. Optionally, said implant has an expansion ratio of at most 1:5 of its diameter before and after inflation.

In an exemplary embodiment of the invention, said implant is adapted to withstand an inflation pressure fo at least 103,42 KPa (15 PSI). Optionally, said implant is adapted to withstand an inflation pressure of at least 344,74 kPa (50 PSI). Optionally, said implant is adapted to withstand an inflation pressure of at least 517,11 kPa (75 PSI). Optionally, said implant is adapted to withstand an inflation pressure of at least 689,48 kPa (100 PSI). Optionally, said implant is adapted to withstand an inflation pressure of at least 1378,95 kPa (200 PSI). Optionally, said implant is adapted to withstand an inflation pressure of at least 2757,9 kPa (400 PSI). Optionally, said implant is adapted to withstand an inflation pressure of at least 4826,33 kPa (700 PSI).

In an exemplary embodiment of the invention, said implant is sterilized. Alternatively or additionally, said implant is provided in a sterile packaging including an indication of a use of said implant.

In an exemplary embodiment of the invention, said implant is adapted for use as a pedicle anchoring screw. Alternatively, said implant is adapted for use as a compression hip nail. Alternatively, said implant is adapted for use as a dynamic hip nail. Alternatively, said implant is adapted for joining fractures of condyles.

### BRIEF DESCRIPTION OF THE FIGURES

Some non-limiting embodiments of the present invention will now be described in the following detailed description of exemplary embodiments of the invention and with reference to the attached drawings, in which same number designations are maintained throughout the figures for each element and in which:
Fig. 1 illustrates two types of implanted pedicle screws, in accordance with some exemplary embodiments of the invention, in a cross-sectional view of a vertebra and the screws;
Figs. 2A-2D are perspective, top, side and side cross-sectional views, respectively, of a pedicle screw with an attached cross-bar rod, in accordance with an exemplary embodiment of the invention;
Fig. 3 illustrates a pedicle screw driver;
Figs. 4A-4F are perspective, top, back, side and two side cross-sectional views, respectively, of a screw inflation system;
Figs. 5A-5D are perspective, back, side and side cross-sectional views of an inline pump and pressure gauge for the screw inflation system of Fig. 4;
Figs. 6A and 6B illustrate expandable proximal femur fixation nails, in accordance with exemplary embodiments of the invention;
Fig. 7 is a schematic side cross-sectional view of an expanding nail, in accordance with an exemplary embodiment of the invention;
Fig. 8 illustrates a hip implant utilizing an expanding femoral insert, in accordance with an exemplary embodiment of the invention;
Fig. 9 illustrates a proximal femur fixation nail utilizing a femoral axial anchor, in accordance with an exemplary embodiment of the invention;
Fig. 10 illustrates three exemplary embodiments of expanding fixation nails, used to stabilize fractures, in accordance with exemplary embodiments of the invention;
Fig. 11A illustrates is a side cross-sectional view of a guided expanding bone implant, in accordance with an exemplary embodiment of the invention; and
Figs 11B-11D are axial cut-through views of the implant of Fig. 11A, showing the relative placement of an inflation lumen and a guiding lumen or slit, in accordance with exemplary embodiments of the invention.

### DETAILED DESCRIPTION OF SOME EMBODIMENTS OF THE INVENTION

The following description covers various types of bone implants that include an expanding portion. In some embodiments of the invention, the expanding portion anchors the bone in cancellus bone, possibly without direct contact with the cortical bone. A potential advantage of such a method of anchoring is that the expansion in the cancellus bone increases the density of the bone and improves the holding power of the implant, even in weak bone. In an exemplary embodiment of the invention, the expansion is by inflation of the expanding portion. However, in some embodiments of the invention other novel features are provided which may be practiced with expanding implants using other expansion mechanisms

Exemplary uses of implants with expanding portions include:
(a) anchors, for example to attach a back straightening rod to a pedicle;
(b) attaching plates to the bone;
(c) supporting bone fixation nails;
(d) supporting artificial joints, such as hips;
(e) bone fixation, for example for cleavage fracture or for fractures in the proximal femur; and/or
(f) bridging between bone fragments.

The description emphasizes two types of implants, bone fixation nails and pedicle screws. For clarity, many exemplary design variations, for example expanding tip designs, materials and expansion mechanisms, are described for only one implant type. However, such variations may also be applied to other implant types (e.g., applied from pedicle screw to bone nail or from bone nail to bone bridge). It should be appreciated that, in exemplary embodiments of the invention, the particular implant design and measurements will depend on the actual function to be carried out by the implant.

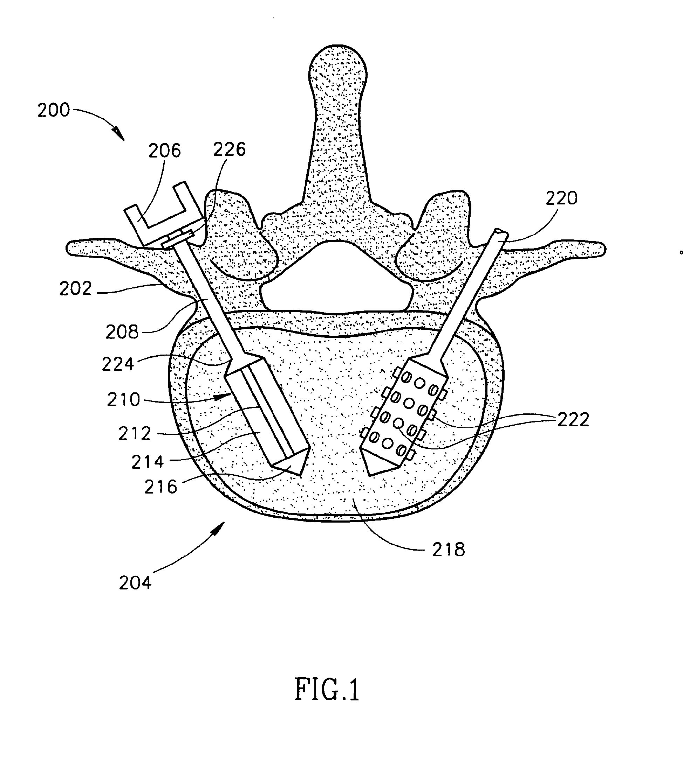

Fig. 1 is a schematic illustration of a pedicle screw 200 (and a partial screw 220, below), implanted through a pedicle 202 of a vertebra 204, in accordance with an exemplary embodiment of the invention. Screw 200 comprises a base 206, used, for example for attaching a cross-bar rod (not shown) for treatment of Scoliosis or Kephosis. The design of base 206 may be any design known in the art and may depend on the type of treatment that the screw is used as an anchor for. A shaft 208 connects base 206 to an expanding tip 210. Shaft 208 is optionally hollow to allow the provision of fluid through it to inflate expandable tip 210.

Other methods of expansion may be used as well. However, it is noted that the body of the vertebra is limited in volume, thus expansion methods that increase the volume of the screw may provide a better anchorage. As will be noted below, screw 200 does not necessarily include any threading. However, the term "screw" is used because of the superficial similarity to pedicle screws. In some embodiments of the invention, the pedicle "screws" may be used as anchors for anchoring attachments to a bone.

Various designs of tip 210 may be used for engaging cancellus bone portion 218 of the vertebral body of vertebra 204 (or the cortex or other parts of the vertebral body). Screw 200 is shown with a tip 210 comprising a thin flexible membrane portion 214 and a plurality (only one shown) of axially extending bars 212. Bars 212 and/or flexible portion 214 are optionally, but not necessarily, heat-treated, so they are less likely to fail during expansion. Alternatively or additionally to straight bars 212, spiraling bars or transaxial bars may be used. Exemplary numbers of bars include, 1, 2, 3, 4 and 5 bars, however, more may be used. Although the bars are not required to extend the length of tip 210, and may be discontinuous, in an exemplary embodiment of the invention, the bars continue from shaft 208 in a straight and contiguous line.

Alternatively or additionally to bar based patterns, a grid pattern may be used. Such non-rib protrusions may be used for pedicle screw 200, as tip 210 is not substantially required to oppose bending strains as is required from intramedullar nails. Typically, the pedicle screw is desired to resist axial retraction forces. However, for some applications, resistance to flexation and/or rotation may be desirable. Such resistance may be provided by the shaft and/or by threading on the screw, it may be provided by friction of membrane 214 with the cancellus bone and/or it may be provided by various bars or protrusion designs.

A partial screw 220 illustrates how the use of a plurality of individual extensions 222 may be used to engage cancellus bone 218. The extensions may be short or long, may be perpendicular to the surface of flexible portion 214 or be angled and/or the extensions may be arranged in a uniform grid as shown or in another arrangement. Additionally, extensions 220 may rotate with respect to portion 214 as tip 210 expands, for example if flexible portion 214 expands in non-radial directions. Exemplary sizes of the extensions (e.g., bars or protrusions) include a diameter of between 0.1 and 3 mm and a height of between 0.1 and 5 mm. The density and texture of the protrusion and portion 214 may also vary, depending, for example, on a desired degree of ingrowth.

A distal end 216 of tip 210 may also expand, or it may have a fixed diameter. Optionally, the expansion of tip 210 extends, in a continuous manner, to distal end 216 and/or an expansion collar 224.

Implantation of screw 200 may include drilling through the cortex of vertebra 204 and/or removing part of cancellus bone 218. Then (or instead) screw 200 is screwed or axially advanced into vertebra 204. Tip 210 and/or shaft 208 are then expanded, to fix screw 200 in place. Optionally, screw 200 includes a thread, for example on tip 210 or on shaft 208, to assist in engaging vertebra 204 and/or to assist in advancing screw 200. Alternatively, a partial threading may be formed only on bars 212 (and not between them, as in complete threading) or the bars themselves (if spiral) act as threading. In an exemplary embodiment of the invention, the expansion of tip 210 is allowed to damage the threading. Such damage is expected if the threading is attached on a radially collapsed tip 210. Alternatively, the threading has pre-formed expansion joints, so that it is not substantially damaged or is damaged in a controlled manner.

If shaft threading is provided, or if the threading is wider than the hole in the cortex, an inner thread may be formed in the cortex, to prevent damage to the bone. Distal end 216 may be self-tapping or may comprise a coring head, to remove a part of the cortex and/or the cancellus bone. It is noted that if tip 210 expands, distal end 216 and/or shaft 208 may have an initial diameter greater than that of tip 210. Exemplary tip diameter expansion ratios include 1:1.2, 1:1.7, 1:2 and 1:4, or intermediate or greater values. In some embodiments, shaft 208 has a texture or protrusion pattern designed to grip the cortex, prevent non-axial motion and/or provide a support for bone ingrowth.

Although a cylindrical expanded tip 210 is shown, the diameter of tip 210 may vary along its length, for example tip 210 may be conical (with either its front or back wider), or tip 210 may have an hour glass shape, be spherical or bulging. Alternatively or additionally, shaft 208 and/or tip 210 may desirably bend during expansion, as a result of the expansion. Alternatively or additionally, distal end 216 may spread apart radially during expansion, for example as two or three split apart segments. An exemplary such design has tip 216 formed of extensions of bars 212, which are split apart from each other by the expansion. Optionally, two inserted screws may meet and engage each other or lock inside vertebra 204.

Alternatively or additionally to tip 210 expanding, shaft 208 may expand, for example along part or all of its length, especially inside the bore in the cortex. Alternatively or additionally, the expansion is at one or both ends of the shaft, to prevent axial motion of the shaft. In an exemplary embodiment of the invention, the expansion of shaft 208 is by screwing a screw into the shaft, where the shaft has a lumen narrower than the width of the screw. In some embodiments of the invention, the same screw can also force a fluid forward into tip 210 and cause its expansion. Alternatively or additionally, also shaft 208 is expanded by the advance of pressurized fluid.

Alternatively or additionally, shaft 208 may be flexible and/or elastic. Alternatively or additionally, shaft 208 may comprise two parts connected by a spring, to allow a small amount of axial motion of base 206 of the screw. A collar 226 (described below) may be fixed or it may also have some freedom of motion.

In an exemplary embodiment of the invention, shaft 208 is contiguous with bars 212, for example the bars being formed by splitting the shaft. Optionally, the bars are heat-treated at expansion collar 224, to allow them to distort without failing. Possibly, bars 212 are heat-treated from inside, for example, through shaft 208, which may be hollow as noted above. Optionally, the heat treatment is sufficient to allow workability (e.g., expansion), while still maintaining resistance to bending and/or twisting. It is noted that the type of forces that are expected to apply to the screw generally determine its structural and/or metallurgical properties. In an exemplary embodiment, screw 200 is formed of titanium or of Nitinol. Exemplary methods of heat-treatment include heating using an electron beam and heating using a laser, for example as described in PCT applications by applicant Discotech Medical Technologies Ltd., serial numbers PCT/IL00/00058, PCT/IL00/00056 and PCT/IL00/00055, the disclosures of which are incorporated herein by reference. These applications also describe other exemplary types of volumetric expansion.

As described above, screw 200 can maintain its location by tip 210 engaging cancellus bone portion 218. Alternatively or additionally, other fixation mechanisms may be used, for example, the cortex of vertebra 204 may be pressure-engaged between expansion collar 224 and a collar 226, which may be thicker than the rest of shaft 208. Alternatively, a part of tip 210 may be inside of the cortex of vertebra 204 and when expanded, engage the cortex directly. Optionally, once the cortex is engaged by tip 210 further expansion of tip 210 is prevented by more distal portions of the tip expanding under the increased strain, with such tips optionally being designed to have less resistance to expansion than the combination of the cortex-tip part and the cortex.

The different types of fixation may limit different types of motion, for example, axial and trans-axial motion, and rotation around the screw axis or transverse to the screw axis. In some implementations, the screw will be desired to prevent all such motions. In others, some motions may be less evenly restricted (e.g., mince less force is expected) or even allowed some freedom of motion. For example, in applications where rotational resistance or bending resistance is desirable, more robust bars 212 may be provided.

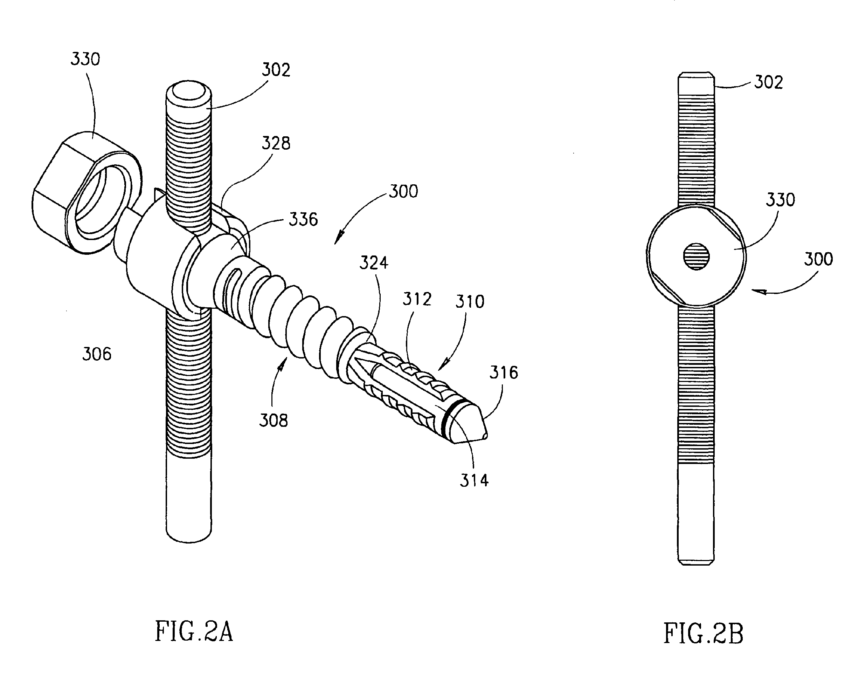

Figs. 2A-2D are perspective, top, side and side cross-sectional views of a pedicle screw 300 with an attached cross-bar 302, in accordance with an exemplary embodiment of the invention. Screw 300 includes a base 306, attached to a shaft 308 via a collar 336, and having an expanding tip 310, with a distal end 316. Tip 310 is formed of a thinner material 314 and surrounded by a plurality of bars 312. Threading is optionally provided on shaft 308 and/or on bars 312. Optionally, the threading, or at least its step and/or width is continuous, or at least matched between the bars and the shaft of the screw.

A cross-bar 302 (e.g., for straightening a spine) is engaged by an external collar 328 that fits on base 306 and is kept in place by a cap 330, for example a screw-on cap. In an exemplary embodiment of the invention, a thin texture, for example a threading or a plurality of thin parallel slots are formed in cross-bar 302, to facilitate engagement by base 306, without requiring an exact positional and/or orientational match between bar 302 and base 306. In use, the screw is inserted, rotated to achieve a desired position for cross-bar 302 (if it exists) and then inflated. Optionally, the screw is partly inflated prior to its rotations. Optionally, the screw is partially deflated if adjustments are required.

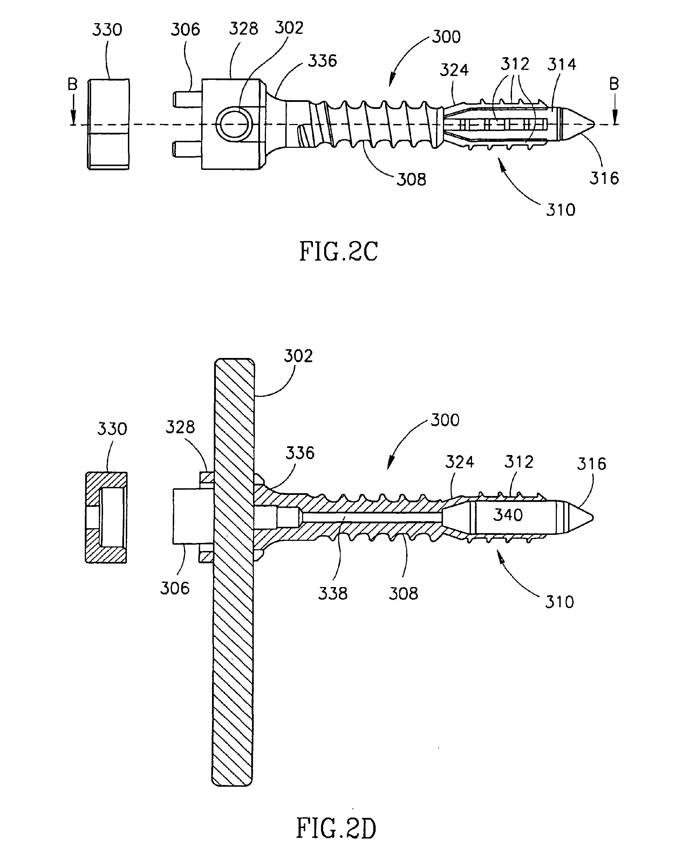

Fig. 2D is a cross-sectional view along a line B-B in screw 300 of Fig. 2C. A lumen 338 connects an opening in screw base 306 to a hollow volume 340 inside head 310.

The number of bars provided in a screw can vary. Possibly, however, bars are provided around the entire circumference of the tip, for example, two, three, four, five or even more bars. The bars may include axial spaces, for example to support expansion of the tip.

Screw 300 may be formed of a single material, for example a plastic, a metal or a composite. Alternatively, the screw is made of several materials, with each part of the screw possibly using a material that matches its function. For example, shaft 308 and base 306 may be formed of Titanium alloy, while head 310 (possibly including bars 312) is formed of Purer titanium, which has better expansion characteristics. Possibly, stainless steel 316LVM, as per ASTM F138-97, is used for the tip and or the shaft of the screw. Optionally, the screw is electro-polished to have a smooth surface.

In an exemplary embodiment of the invention, a pedicle screw has the following priorities: total length comprising between 40 and 60 mm for threading plus a 17mm base; an inflatable tip length of half the threaded length, included in the threaded length; an inner thread diameter of d₅=4.5mm, based on an ISO 5835:1991 (e) table 4 type HB 6.5 thread; a 2.75 mm pitch for the thread. The threading is separated into two part: one distal part with 5 turns and one proximal part with 2.25 turns and a tapered angle of 12°. The thread diameter is 6.5mm OD and 4.5mm ID. The diameter of the inflatable tip is between 4.5 (un-inflated) and 7.1 (inflated). The top cap diameter is 12.6 and its thread is M9*1mm. The bars on the inflatable tip have a width of 1.3mm and a height of 1.2 mm. The membrane has an elongation of between 40% and 50%. The cross-bar rod has a diameter of 4.8 mm. Other exemplary inflatable tip bar dimensions are a width and/or height of 0.1mm, 0.4mm, 1mm, 2mm, 3mm or any intermediate or greater number. The cross-sectional profile of the bars, which may be constant or may vary, may be, for example, rectangular, circular, a half circle, a triangle, a trapezoid or an inverted trapezoid.

These dimensions may vary for different types of screw and/or for different sized patients. For example, a length of the screw can be, for example, 10mm, 20mm, 40mm, 70mm, 100mm, 150mm or any intermediate length. A diameter of the screw (not including the thread) can be, for example, 2mm, 4mm, 6mm, 10mm, 15mm, or any intermediate or greater diameter a depth of the threads can be for example, 0.5mm, 1mm, 3mm, 5mm or any intermediate or greater value. the elongation property of the membrane can be, for example, 10%, 12%, 15%, 20%, 27%, 35%, 40% or other intermediate or greater percentiles. An inflated diameter can be, for example, 4mm, 7mm, 10mm, 15mm or any intermediate or greater diameter. The volume displaced by the expansion can be, for example, 0.1cc, 0.5cc, 1cc, 2cc or any intermediate, smaller or greater volume. An angle defined between the shaft and the inflated head, can be, for example, 160°, 140°, 110° or even a sharper angle.

Another exemplary measurements is, a ratio between the length of the inflating part and the total length of the shaft, which may be, for example, 70%, 60%, 50%, 35%, 20%, 10%, or any intermediate or smaller value. A related measure is the position of the center of the inflated part on the shaft, which may be, for example, at one end of the shaft, less than 10%, 20%, 30% or 40% along the length of the shaft, as measured from either end, or even at the shaft center. Some implants are characterized by the ratio between the length of the implant and its shaft diameter, which can be, for example, 3:1 (short fat nail), 5:1, 10:1, 20:1 or intermediate or greater or smaller ratios.

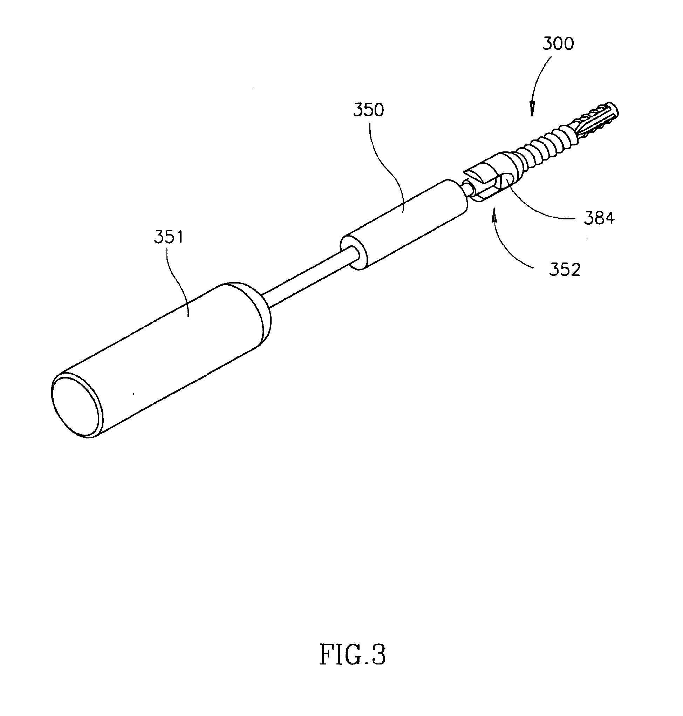

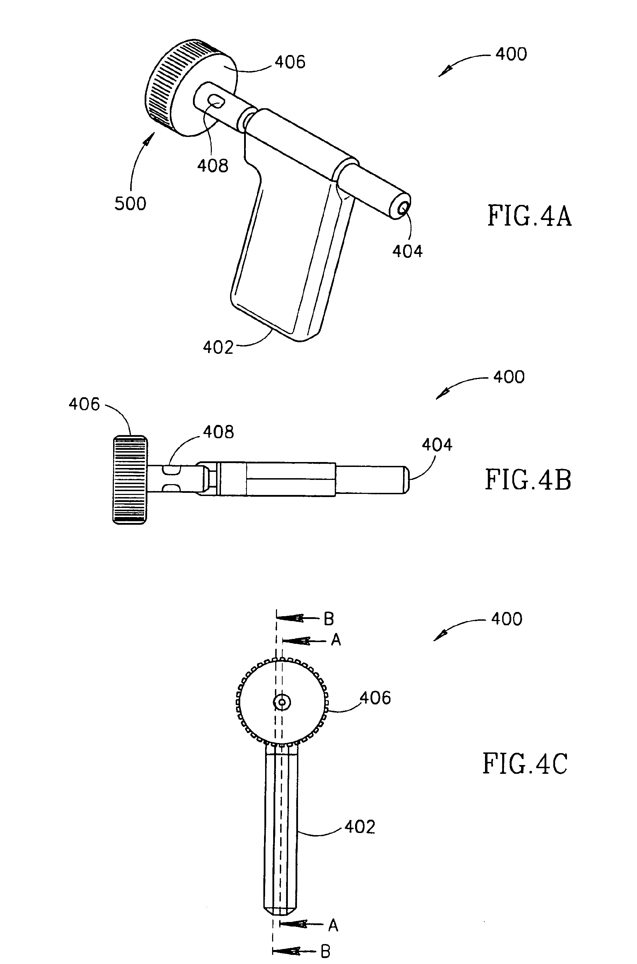

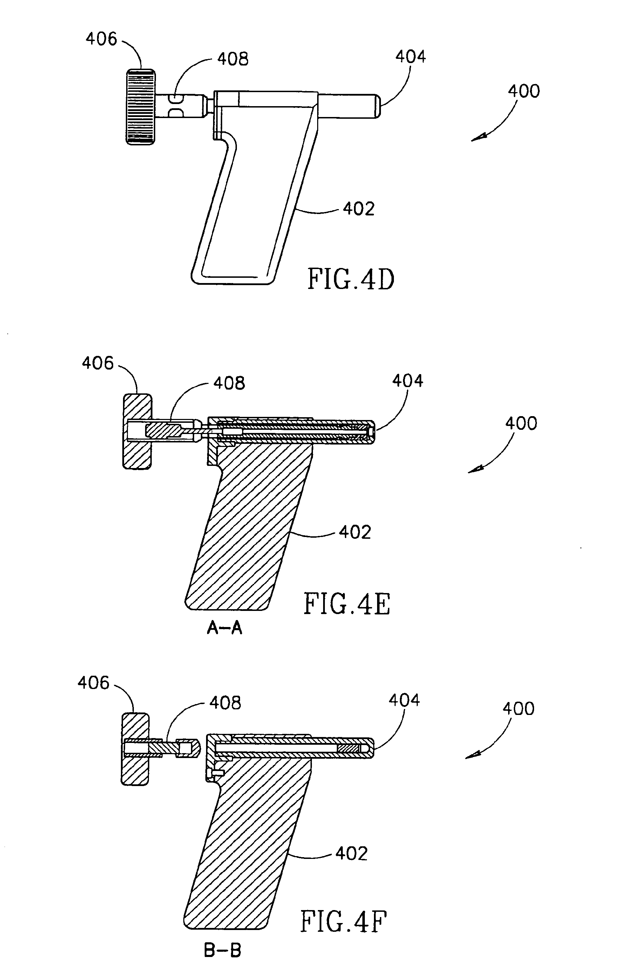

Fig. 3 illustrates a pedicle screw driver 350, in accordance with an exemplary embodiment of the invention. Screw driver 350 generally comprises a handle 351 and a tip 352. Optionally, tip 352 includes a fluid pressure outlet adapted to transfer pressurized fluid from an opening 404 (Fig. 4) to the inner lumen of the screw. In an exemplary embodiment of the invention, tip 352 includes a wide element 354 that engages base 206 of the screw. Optionally, tip 352 includes an additional axial extension (not shown) that engages a hollow formed in base 206.

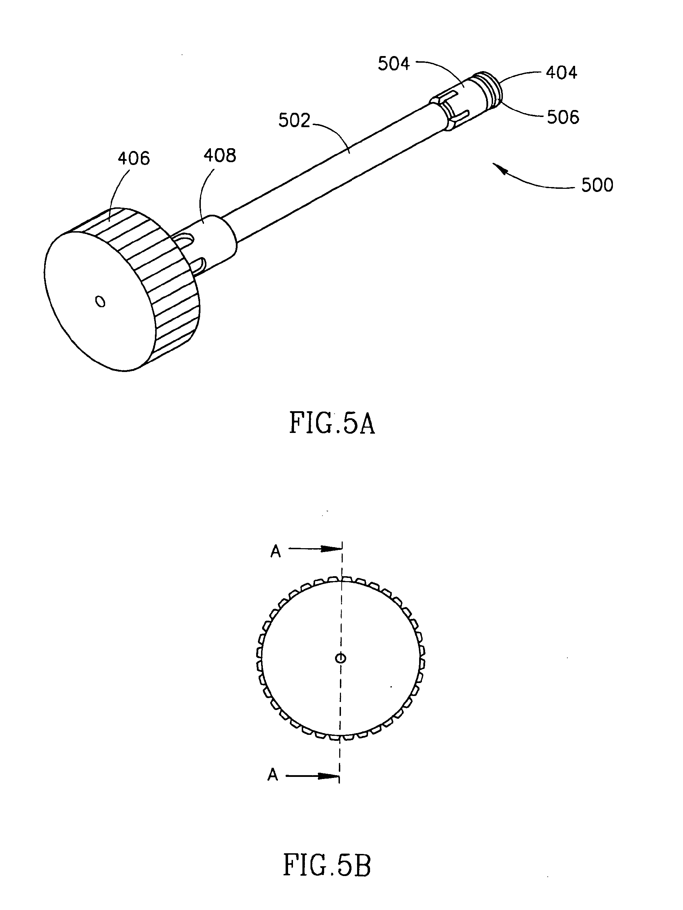

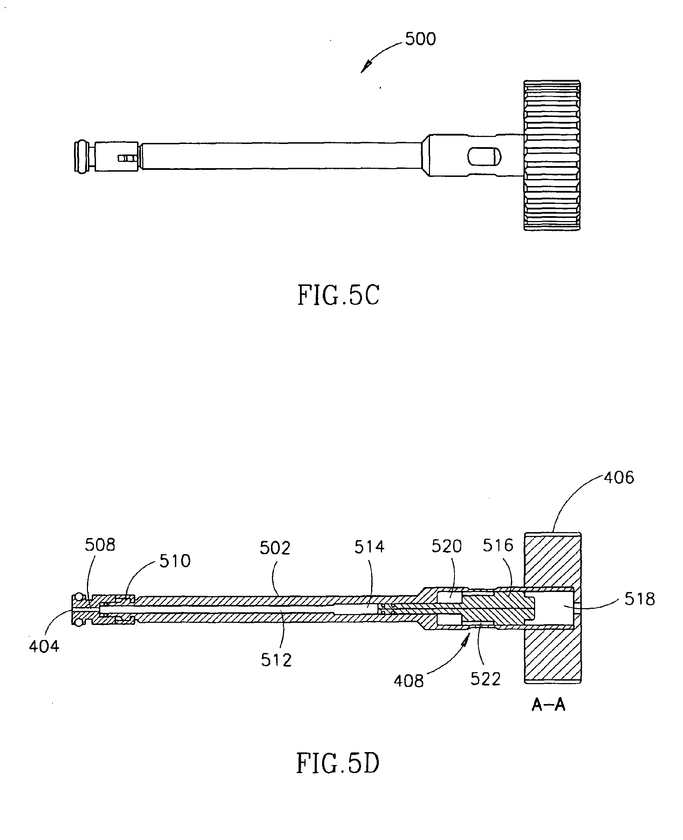

Figs. 4A-4F are perspective, top, back, side and two side cross-sectional views of an inflation system 400, in accordance with an exemplary embodiment of the invention. System 400 generally comprises a handle 402, a tip 404 from which a pressure tube (optionally flexible and not shown) extends to base 306 of screw 300 (Fig. 2) and a pump 500. In an exemplary embodiment of the invention, pump 500 is a screw type pump rotated by a knob 406, which pump advances a fluid by advancing a threaded piston into a fluid reservoir. In an exemplary embodiment of the invention, a pressure indicator 408 is provided on a shaft of pump 500, as will be described in more detail in Fig. 5, below.

In use, when pump 406 is rotated, a shaft connected to the pump is advanced into a fluid reservoir (shown in Fig. 5), forcing the fluid into screw 300, so that it inflates.

Figs. 5A-5D are perspective, back, side and side cross-sectional views of pump 500, in accordance with an exemplary embodiment of the invention. Exemplary pump 500 comprises a knob 406 mounted on a long shaft 502, terminating in a reservoir 504. The end of the reservoir is shown as point 404. A ring 506 may be provided on the reservoir, to prevent it from turning and/or axially exiting from system 400 (Fig. 4). Alternatively or additionally, other fastening means may be used.

As shown in the cross-sectional of Fig. 5D, a lumen is defined between a reservoir lumen 508 via a shaft lumen 512 to a gauge lumen 514. A marker plug 516 plugs the other end of gauge lumen 514, optionally using one or more O-rings to prevent leakage of the fluid from lumen 514 past the plug. However, as pressure in gauge lumen 514 increases, the plug is pushed away, towards the knob. A counter-force preventing plug 516 from moving may be provided, for example by providing pressurized fluid in a lumen 518 in the other side of plug 516 from gauge lumen 514, or by plug 516 forming a vacuum in a plug lumen 526. Alternatively or additionally, a spring or an elastic molding may be used.

In an exemplary embodiment of the invention, one or more slots 408 are formed in a proximal portion of shaft 502, near knob 406. Marked regions 522 on plug 516 come into alignment with slots 408, as the pressure increase, thereby indicating the pressure. It is noted that the above pressure indication system may be cheaper, more rugged and/or easier to read than standard dial-type pressure gauges, especially if high pressures, such as 689,48 kPa (100 PSI) are being measured.

In some embodiments, the tip is inflated using a fluid, such as silicon gel or saline solution or other fluid which is stored in the shaft of the screw and advanced into the tip by rotating a screw in the shaft or by applying pressure against a base of the shaft, thereby reducing the free volume in the shaft. In some embodiments of the invention, the working fluid hardens, for example as result of heat or time (chemical reaction). Alternatively, if the deformation of screw 300 is plastic, in some embodiments of the invention, the fluid can be removed.

In some embodiments of the invention, the screw includes a vent, to prevent escape of the fluid. Alternatively, no vent is provided, and the hole is sealed or the procedure is delayed until the working fluid hardens. Alternatively, a stopper is used to plug the lumen entering the screw, or the screw lumen includes a tubular section that is crimped shut.

In some embodiments of the invention, non-inflation type expansion mechanisms are used, for example, an inner portion of shaft 308 can advance and expand tip 310 during its advance. Alternatively or additionally, tip 310 is super elastic or shape-memory and expands when a restraint (e.g., an outer tube) is removed or heat (e.g., body heat) is supplied. Alternatively or additionally, distal end 316 is attached to a rod, a screw or cable, which, when pulled, retracts end 316 into tip 310, causing tip 310 to expand.

Other exemplary types of expansion mechanisms may be found for example in the above IB PCT application. It should be noted however, that the different expansion types are generally not equivalent with respect to their mechanical properties, mechanical effect on the bone, long term healing effect on the bone, bone ingrowth, ease of removal, amount and type of movement of the implant during the expansion and/or suitability for certain applications.

In some embodiments of the invention, screw 310 is removed after a time. To facilitate such removal, tip 310 or bars 312 may be formed of a bio-absorbable material. Alternatively or additionally, screw 310 is deflated. Alternatively or additionally, the threading is used to assist in screw removal. In some embodiments of the invention, removal is not desirable and the texture and/or geometry of the screw tip and/or shaft are adapted to encourage bone ingrowth.

In an exemplary embodiment of the invention, a complete kit is provided, for example in a sterilized package. Such a kit may include, for example, one or more screws, a screw driver, for rotating the screw, one or more rods (for spinal fixation or spinal straightening applications), an inflation system and/or a suitable drill bit. Not all the parts need to be provided in a same kit. In an exemplary embodiment of the invention, suggested use for the screw is marked on the kit, for example a range of angles and/or a bone and/or fracture types for which such a screw (or other implants described herein) are suitable for. Alternatively or additionally, a table or calculator may be provided, for example on paper or in electronic form, for matching a suitable implant or range of implant dimensions to a particular application.

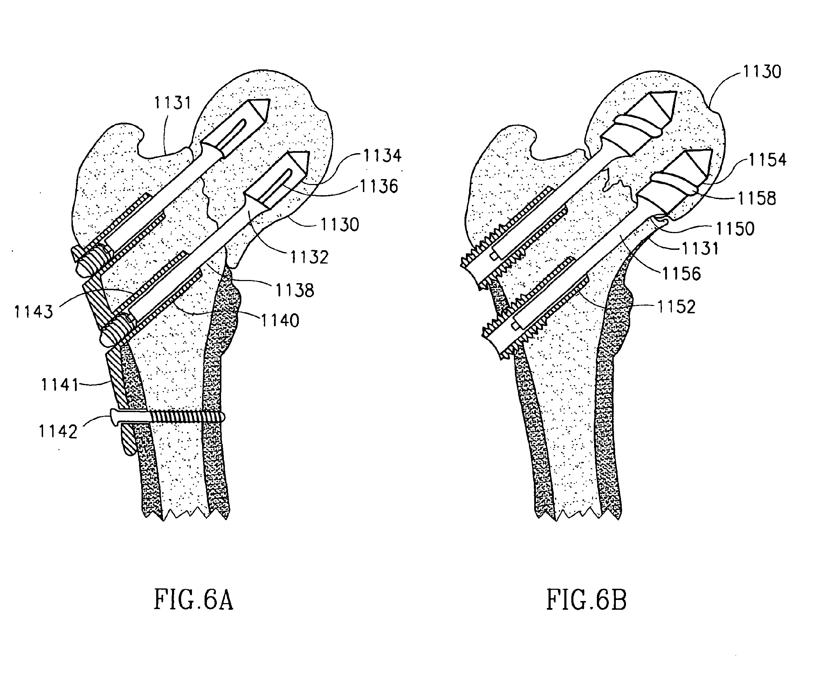

Figs. 6A and 6B illustrate expandable femoral neck fixation nails, in accordance with exemplary embodiments of the invention. Fig. 6A illustrates a compressive hip nail 1132 (two shown) that has an expandable tip 1134 embedded in a femur head 1130 and has a shaft 1138 attached into a base 1140 in a second part 1131 of the proximal femur. Thus, the hip is held together by compression between the nail 1132 and base 1140. Optionally, base 1140 comprises also an external plate 1141 and, further optionally, one or more optional second nails 1142 fixing the plate to the femur. Optionally, base 1140 is attached to nail 1132 using an inner thread on a shaft 1143 of base 1140. Alternatively, other attachment methods, such as using a bolt, or a base of nail 1132 being too wide for shaft 1143, may be provided for attaching plate 1141 to femur part 1131. Possibly, no plate is provided.

In an exemplary embodiment of the invention, tip 1134 is an expandable tip, which is expanded, after tip 1134 is inserted into femur head 1130. Optionally, one or more bars 1136 (or other protrusions), for example three, are located on the circumference of tip 1134, for example using patterns as described below. Bars 1136 optionally hold tip 1134 in place and prevent nail 1132 from moving within the hip. In some cases, bar 1136 is used to stiffen tip 1134 and/or to push aside spongy bone, during the insertion of tip 1134 into femur head 1130. In other cases, bar 1136 and/or the other protrusions are provided and/or machined to encourage bone ingrowth.

Alternatively or additionally to having bars 1136 on tip 1134, non-rib protrusion patterns, such as a grid or protruding spikes, are placed on tip 1134. Such non-rib protrusions may be used in hip nail 1132, in which tip 1134 is not substantially required to oppose bending strains as is required from intramedullar nails. Alternatively or additionally, transaxial bars are provided on head 1134. Possibly, a greater internal pressure is required from hip nails, to aid in transferring pressure from the surrounding bone to the nail, without the nail collapsing.

Fig. 6B illustrates a dynamic hip nail 1150, comprising a tip 1154 and a shaft 1156. In dynamic nail 1150, shaft 1156 is not rigidly fixed to a base 1152. Instead, shaft 1156 is restrained to move along an axis of a tube portion of base 1152. Optionally, expanding tip 1154 comprises one or more spiral-arranged protrusion bar 1158. Alternatively, tip 1134 with bars 1136 and/or any other expanding tip may be used in nail 1150. Optionally, base 1152 is attached to the bone by expansion of one or expandable areas thereon (not shown). Alternatively, other means, such as threading, or a separate fastener, may be used.

In some embodiments, the tip is inflated using a fluid, such as silicon gel which is stored in the shaft of the nail and advanced into the tip by rotating a screw in the shaft of the nail, thereby reducing the free volume in the shaft. Alternatively or additionally, an external pump is used to increase the pressure and/or provide fluid, in the nail.

In some exemplary embodiments of the present invention, tips 1134 and/or 1154 are partially or entirely heat-treated to allow inflation of the tips without breakage. In a exemplary embodiment of the present invention, parts of bars 1136 and/or 1158 are also partially or entirely annealed, for example at their point of maximum expansion. Nails similar to nails 1132 and 1150 or at least utilizing similar tips, may also be used in other parts of the body in place of other implants such as regular prior art nails and screws.

In weak bones, especially osteoporosic bones, such as common in older patients, expandable tips are generally expected to yield better holding power than threaded nails or screws and/or shaped rods. Also, by periodically increasing the inflation, a loose nail can be tightened. Alternatively or additionally, by reducing the inflation, the nail may be loosened, to assist retraction. In some embodiments of the invention, the expansion of the head is past the elastic limit. However, this is not required and the head may be expanded to within an elastic, super-elastic or shape-memory limit (if relevant).

It is noted, however, that in some cases it may be advantageous to allow the grip of tip 1134 within femur head 1130, to weaken after a period of time. In such cases bar 1136 and/or 1158 may be heat-treated accordingly (e.g., to be softer or more elastic) to allow timely retraction of nail 1132 or 1150. Alternatively or additionally, to using heat-treatment to improve workability of a materials, other methods may be used to modify the properties of different parts of the implant, for example, cold or hot working, annealing, selecting suitable and different materials and/or chemical treatment.

In Figs. 6A and 6B two nails are shown. In some embodiments of the invention, only one nail is used to stabilize a femoral neck fracture. Two or more nails may be used, for example, to increase the strength of the connection or to prevent rotation. The plane defined by the nails may be coplanar with the femur axis or perpendicular to it, or at a different angle to it. Optionally, the two nails are not parallel to each other, so that the angling further prevents motion of the bone parts. Alternatively or additionally, one of the nails is not an expanding type nail. Alternatively or additionally, the different nails may have different patterns on their expanding heads. Alternatively or additionally, the pattern may be reversed, for example on two nails 1150, the spirals of bars 1158 may be in opposite direction. Such opposite pattern directions may also are provided in other nail designs, for example where the nail is threaded, on the shaft and or on the head. In nail 1150, the threading and the spiraling in a single nail may be in opposite directions.

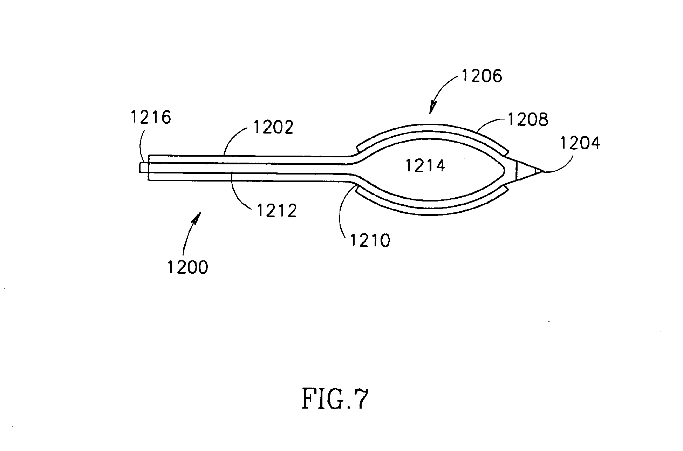

Fig. 7 is a schematic side cross-sectional view of an expanding nail 1200, in accordance with an exemplary embodiment of the invention.

Nail 1200 comprises a shaft 1202 and an expanding tip 1206. Optionally, the tip is capped at its distal end using a point cap 1204, which may be expanding (distorted by head 1206, when it expands) or non-expanding. Point cap 1204 may be, for example, sharp or blunt and/or self tapping. In some embodiments, nail 1200 is inserted into a pre-drilled hole. Alternatively, nail 1200 drills its own hole or is screwed or pushed in.

A lumen 1212 is defined in shaft 1202 and connects to a larger lumen 1214 inside head 1206. In an exemplary embodiment of the invention, head 1206 is formed of a thin membrane covering 1210, for example metal or rubber, on which a plurality of protrusions, such as bars 1208 are provided. When nail 1200 is inflated, a fluid is provided at a vent 1216, which may include a valve or simply be an opening that is sealed after used, for example, by crimping. The provided fluid travels through lumen 1212, enters lumen 1214 and thus inflates tip 1206. As noted above, other parts of nail 1200 may be expandable and may utilize similar structure, for example, part or all of shaft 1202, which may also be formed of a membrane and bars, at least for some of its length. Alternatively, shaft 1202 has thick walls. Alternatively or additionally, shaft 1202 may be flexible and/or even elastic. Threads may be provided on some or all of shaft 1202 and/or head 1206.

During delivery, head 1206 is collapsed, for example plastically, elastically or super elastically. Thus, in some embodiments, no pressurized fluid is required for expansion. Alternatively or additionally, to a fluid, a hardening polymer may be provided. In some respects the design of nail 1200 is similar to that of a pedicle nail as described above and an intramedullar nail as described in the above referenced 1998 PCT application.

Nail 1200 may be formed of various materials, including metal and plastic. The nail may be formed of a single material or of several materials. In an exemplary embodiment of the invention, the nail is formed of a titanium alloy, while the expanding head (or at least the membrane) is formed of pure titanium, which has better elongation characteristics. Alternatively or additionally, the nail, or at least part of it are formed of stainless steel.

Tips 1134 and 1154 (Fig. 6) are shown as having the general shape of a cylinder. However, in some embodiments of the invention, other head shapes are used, for example heads having a different (greater or smaller) surface to volume ratio or heads having a directional bias, which may be suitable for counteracting forces in certain directions. In one example, tip 1134, when expanded has a flattened rectangle cross-section. Optionally, the expanded configuration is concave, at least on one side. Alternatively or additionally, the tip geometry may vary along the axial direction of the nail, for example by bars 1136 preventing expansion at certain parts of the nail or by providing a constricted metal membrane 1210 (Fig. 7) at those points. Alternatively or additionally, a restraining ring may be mounted on the nail.

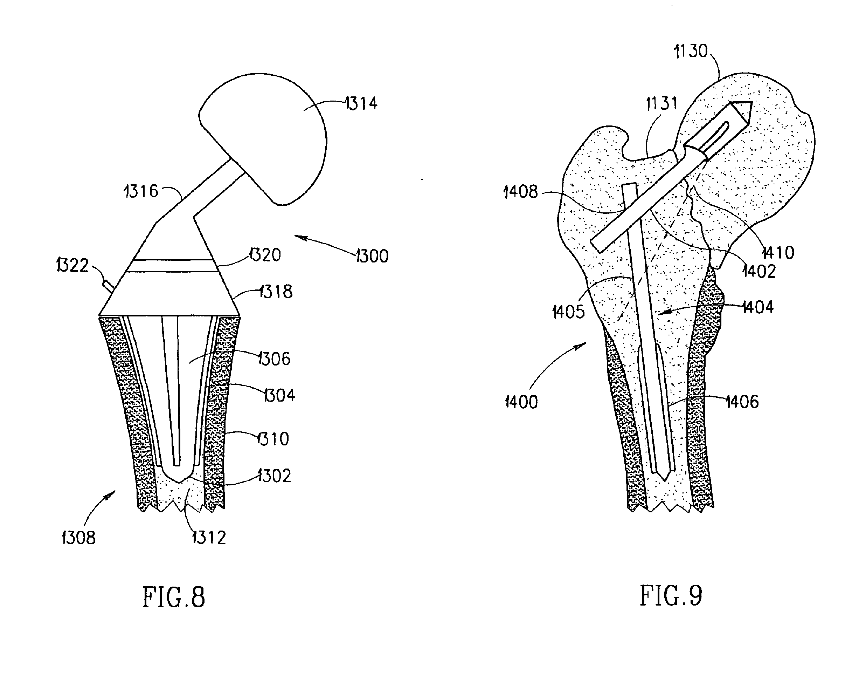

Another example of an inflatable implant is a hip implant, where a portion that engages the femur can be an inflating shaft. Fig. 8 illustrates a hip implant 1300 utilizing an expanding femoral insert 1302, in accordance with an exemplary embodiment of the invention. A femur 1308 has at least part of its medullar channel 1312 reamed out. Insert 1302 is inserted into the reamed channel and expanded, so that it grips an inside surface of a cortical section 1310 of femur 1308. In an exemplary embodiment of the invention insert 1302 is formed of a metal membrane 1306 having a plurality of protrusions, such as a plurality of axial bars 1304, formed on it or attached to it.

A base 1318 is preferably fixed to insert 1302, and a ball hinge 1314 (or other hip parts) are mounted on an extension 1316 which is also attached to base 1318. In some embodiments of the invention, hinge 1314 is an original femur head and implant 1310 is used to replace a fractured femoral neck. A vent 1322 is optionally provided for initial and/or periodic inflation and/or deflation of insert 1302.

Various exterior finishes may be provided on insert 1302, instead of bars 1304 as shown. In one embodiment of the invention, the membrane is clear of bars. Optionally, an internal axial structural element is provided to prevent tearing of membrane 1306. Alternatively or additionally, a pattern of protrusions, for example, spiral, transaxial, and/or direction-less protrusions, such as a grid, are provided on the membrane. Alternatively or additionally, the surface of membrane 1302 is machined to enhance or retard tissue ingrowth. Alternatively or additionally, it may be coated with an ingrowth enhancer or retarder. Alternatively or additionally, a mesh element is provided between insert 1302 and cortex 1310, for example, to improve traction.

In an exemplary embodiment of the invention, a tissue adhesive, bone cement and/or bone chips are provided between insert 1302 and cortex 1310. The material may be provided, for example, by flow along bars 1304 or through a plurality of opening in insert 1302 (not shown), for example the flow being inside bars 1304 or between the bars and the membrane. Optionally, excess adhesive leaks out through the top of insert 1302, or through a vent in base 1318 (not shown), which may also be used for providing the adhesive.

In some types of implants, the implant is removed and/or replaced periodically. In an exemplary embodiment of the invention, the removal of insert 1302 is enhanced by deflation of the insert. Alternatively or additionally, a coupling 1320 is provided between the insert and the hip joint part, so only one of the two needs to be removed and/or to assist removal of both parts.

Fig. 9 shows a different type of device that utilizes an anchor portion and a second portion. Fig. 9 illustrates a fixation device 1400 including a femoral neck fixation nail 1402 and a femoral axial anchor 1404, in accordance with an exemplary embodiment of the invention femoral insert 1404 includes a shaft 1405 and an expanding portion 1406, which may utilize a similar design to that of expanding intra-medullar nails and/or hip implant insert 1302 (Fig. 8). Anchor 1404 may contact the cortical bone surface. Alternatively, as shown in the Figure, there may be no contact between the anchor and the cortex. In implants where such contact is desired, the anchor may include protrusions or other features for engaging the cortical surface and prevent axial retraction.

Nail 1402, which may utilize the design of the nails shown in Figs. 6A, 6B and 7, is coupled to shaft 1405 at a coupler 1408. In some embodiments of the invention, the coupler is simply a hole in shaft 1405. Alternatively or additionally, a connector, which may be a rigid connector or a flexible connector is provided. In some embodiments of the invention, some degrees of motion are allowed, for example, axial motion (along nail 1402). In this embodiment, coupler 1402 may be similar in design to shaft 1152 of Fig. 6B, albeit possibly shorter.

In an exemplary method of deploying device 1400, two narrow holes are drilled in the femur, one at least partly along the proposed path of nail 1402 and one at least partly along the proposed path of axial anchor 1404. The two parts of device 1400 are then inserted, inflated for provide fixation and then coupled. The order of insertion may depend on the geometry of the device parts. The inflation of the two device parts may be simultaneous, in series or partly interleaved in time and the coupling may be performed before, after or even during inflation. In an exemplary embodiment of the invention, a coupling element or a coupling action (e.g., rotating a bolt) is provided along the hole drilled for nail 1402. Alternatively or additionally, the coupling is provided along the hole drilled for anchor 1404. In some embodiments of the invention, the coupler is a bio-compatible polymer injected into the coupling area, rather than a pre-formed metal or plastic part. Alternatively or additionally, inflation of the shaft of the anchor or of the nail is used to complete the coupling.

In some embodiments of the invention, a support strut (shown as dotted line 1410) is provided through a second hole in shaft 1405 and to engage or contact nail 1402, Alternatively, such a strut may be completely outside the femur or attached to one part of the device inside the femur and to another part outside the femur, for example, using a nail. Contact between the strut and the nail and/or the anchor may not be required, for example if the strut is expandable, or can transfer the strain from and to the bone without coupling to the nail and anchor. Contact between the different elements may still be desirable, however, it is not required.

Alternatively, two nails may interlock, for example a through hole being defined in one nail, for the passage of the other nail.

In an alternative exemplary embodiment of the invention, the femoral neck is held using one or more plates on the outside of the bone, which enter the bone and engage an expanding axial anchor, such as anchor 1404.

Although only one nail 1402 is shown, in some embodiments, two or more nails, which do or do not couple to shaft 1405, are used.

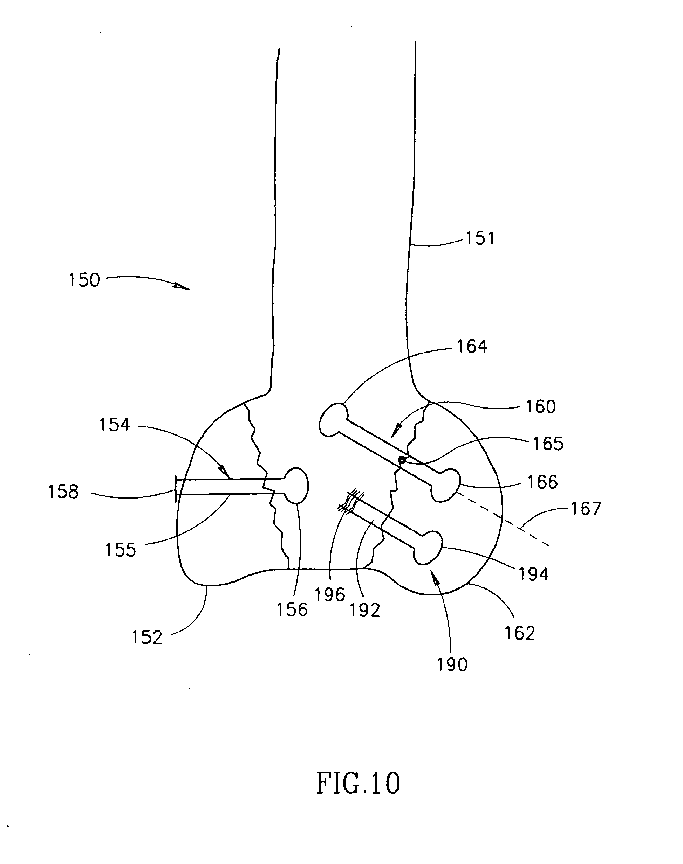

Expanding implants may be used for stabilizing other types of fractures as well, for example, cleavage type breaks in bone ends (separation of condyles), for example in the femur and tibia. Fig. 10 illustrates three exemplary embodiments of expanding fixation nails, used to stabilize fractures, in accordance with exemplary embodiments of the invention. A bone 150 having a shaft portion 151 and two condyles 152 and 162 is shown fractured such that the two condoles are separated. As well known in the art, various degrees of separation may require a fixation nail, for example, small, large or complete cracks or a complete separation between the two bone parts.

A nail 154 is a first exemplary nail, having a shaft 155, an expanding tip 156 and a base 158, for abutting bone 150, either directly, or through a plate, for example for contact pressure distribution or for attachment to the bone.

A nail 160 is a second exemplary nail including a shaft 161 connecting an expanding tip 164 and an expanding head 166. The two expanding parts may use a same expansion mechanism or different expansion mechanism.

In an exemplary embodiment of the invention, expanding parts 164 and 166 are inflatable and share a same lumen. In one exemplary embodiment of the invention, the two expanding parts expand substantially simultaneously in response to the provided pressurized fluid. Alternatively, one of the expanding parts is made stiffer, so that it starts expanding only after the other part has already started, or even finished, expanding.

Alternatively, the two expanding parts use separate inflation lumens. The two lumens may be connect the inflatable parts to opposite sides of the nail, one or both may be on the side of the nail, or they connect to a same side of the nail, for example, they may pass alongside each other.

In an exemplary embodiment of the invention, prior to or after inserting the nail, missing parts of the bone are reconstructed using bone chips and/or bone cement. In some embodiments of the invention the shaft (e.g., 155 or 161) are made thin, so as not to damage small or thin bone sections which are transfixed by the nail. Optionally, threading is provided on some part of the nail, for example, on one or both of the expanding parts and/or on the shaft.

The nail may be provided through a bore formed in the bone, indicated, for example by a dotted line 167 for nail 160. Alternatively, the two parts of the bone are closed on the nail. The inflation of the nail may be through bore 167, or possibly through a side vent 165 of the nail, which type of vent may be used if forming bore 167 is not desirable.

A third exemplary nail 190, has a shaft 12, one expanding head 194 and one threaded head 196. This type of nail combines two different types of bone anchoring, expansion and threading.

In an exemplary embodiment of the invention, the shaft is flexible and/or axially stretchable, thus allowing some freedom of motion between the bone parts, for example a small amount to enhance healing, or a larger amount, to replace ligament function. Alternatively or additionally, an expanding part of the nail may be use to hold a tendon or a replacement tendon in place against a bone.

Although expansion of end regions of a nail are described, in some embodiments, expansion of a central portion of the nail is desired, with or without expansion of one or both the nail ends. In one example, both ends of the nail exit the bone and are used for attachment of plates thereto and the center of the nail is used for anchoring.

The above expanding mechanisms may also be used in cortical bone. One advantage of inflation for cortical bone fixation, is that an inflated part can automatically conform to the geometry of the hollow in a cortex in which it is placed. For example, when one part of the inflating part contacts the surrounding cortex, further expansion of the part is delayed, due to the increased local resistance to expansion, until all the rest of the inflatable part contacts the cortex.

In some embodiments of the invention, especially for bone fracture repair, such as shown in Fig. 10, it may be desirable to form the nail out of a bio-absorbable material, for example a plastic matrix. Possibly, the material (or texture) also enhance bone ingrowth. In such a bio-absorbable device, the working fluid is also preferably bio-absorbable, or at least bio-compatible. Possibly, a hardening working fluid is used, so that when the integrity of the nail is compromised by bio-absorption, the nail does not collapse at once from the loss of fluid. The degradation time of the nail may be selected to match expected tissue healing rates.

As indicated above, the particular design of an implant may vary, depending on the function of the implant and the properties of the bone in the particular patient and/or expected effect of the implant geometry on the healing of the wound caused by the implantation or that which the implant is intended to help. Following are various exemplary design decisions which may be made for some applications. However, these example should not be taken as limiting a suitable design to only the example described.

In one example, the implant is designated to resist axial retraction (e.g., simple anchoring). This may affect, for example, the degree of expansion of the tip, the alignment direction of the bars and the angle between the inflated portion and the shaft. In some such implants, no bars are required. In another example, an important purpose of an implant may be to increase the density of the bone at the locality. This can be achieved by increasing the volume of the expanding portion. In another example, the implant must resist bending, torsional forces and/or lateral movement. In this example, axial bars may be suitable. In another example, the implant transfers strain from one part of a bone to another, thus, stronger bars may be useful. Contiguous bars (also the between inflatable portion and the shaft) may be useful when the implant provides a resistance to axial extension. Transaxial bars may be useful to provide compression between two bone parts. The geometry of the implant may also be used to provide flexibility or elasticity in a certain direction, for example spiral arranged bars may provide some degree of axial elasticity.

In an exemplary embodiment of the invention, the arrangement of protrusions on the expanding portion can be selected to provide zero, positive or negative axial motion during inflation. In one example, using axial bars, no axial motion is expected. If transaxial bars are provided, the expansion of the membrane between the shaft and the first bar and between the bars, may allow some motion. Angled protrusions may urge the inflating parts either towards or away from the rest of the implant. In some embodiments, the membrane is folded in a radial direction, during deployment. Alternatively or additionally, the membrane may be folded in an axial direction, for example, to provide for some axial motion. Another consideration relating to the arrangement of the protrusions and the surface finish is whether the implant is to be removed or is permanent. Smooth implants tend to loosen after a while, which may be a benefit if the implant is to be removed.

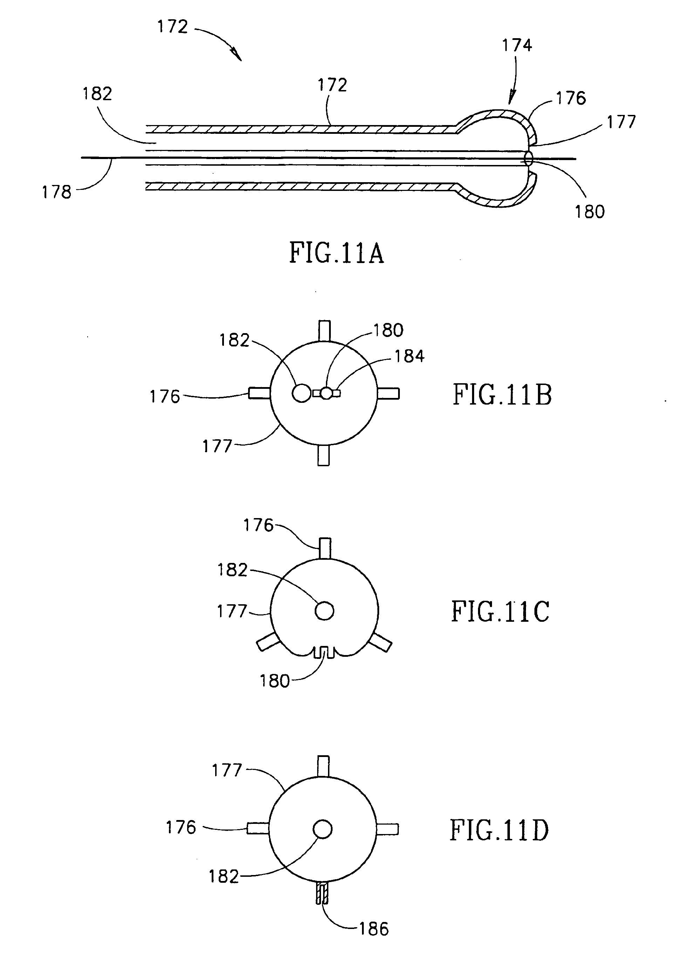

Fig. 11A illustrates is a side cross-sectional view of a guided expanding bone implant 170, in accordance with an exemplary embodiment of the invention. The implant comprises a non-expanding shaft 172 and an expanding tip (or other part of the implant) 174, which is for example, adapted to anchor in cancellus bone. Tip 174, for example, includes a membrane 177 and a plurality of bars 176, for example using any of the designs described above. Threading may be provided in some embodiments of the invention. In Fig. 11A, two co-axial lumens are provided, an inner (or outer) lumen 178 for a guide wire, guide strip or guide rod 178 and an outer lumen 182 for providing pressurized fluid. In an exemplary embodiment of the invention, the walls between the two lumens are made stiff enough to resist collapsing when lumen 182 is pressurized. Alternatively, the guide wire is retracted prior to pressurizing lumen 182.

Figs 11B-11D are axial cut-through views of the implant of Fig. 11A, showing the relative placement of an inflation lumen and a guiding lumen or slit, in accordance with exemplary embodiments of the invention. In Fig. 11B, the two lumens 182 and 180 are side by side, with an optional stiffening 184 provided to prevent collapsing of lumen 180. In Fig. 11C, inflation lumen 182 is axial, while a guide slot 180 is provided along the side of implant 170.

Possibly, the slot or lumen is provided only along part of the implant, for example along shaft 172. Alternatively or additionally, only an open slot is provided for tip 174. Possibly, slot 180 has a wider channel than its opening (e.g., an upside-down triangle profile), to prevent a guide wire 178 from falling off.

In Fig. 11D, a slot 186 is formed in one of the bars 176, possibly a wider bar.

In some embodiments of the invention, the guide wire or rod is curved, to assist in insertion of a nail with a curved shaft. Alternatively or additionally, especially when two or more nail are inserted, a framework is provided the framework is fixed to the bone and the guide wire(s) are inserted into the bone and maintained in their correct positions by the framework.

It will be appreciated that the above described apparatus and methods of expandable inserts may be varied in many ways. In addition, a multiplicity of various features, both of methods and of devices have been described. It should be appreciated that different features may be combined in different ways. In particular, not all the features shown above in a particular embodiment are necessary in every similar described embodiment of the invention. Further, combinations of the above features are also considered to be within the scope of some exemplary embodiments of the invention. When used in the following claims or in the text above, the terms "comprises", "comprising", "includes", "including" or the like mean "including but not limited to".

## Claims

1. An implant (200, 300) adapted to anchor in cancellus bone, comprising:
a shaft (208, 308) having a longitudinal axis and defining an inflation lumen (338) therethrough; and
at least one inflatable anchor portion including a thin inflatable flexible membrane and connected to said lumen, whereby inflation of said anchor portion causes said implant to engage said cancellus bone, wherein said anchor portion includes a plurality of protrusions (222, 1158) extending from said membrane perpendicular or at an angle to a surface of the membrane and adapted to engage cancellus bone, wherein said anchor portion is at a distal or proximal tip (216, 316) of said shaft or is at a center portion of said shaft, and wherein the anchor portion comprises less than 60% of a length of said implant.

2. An implant according to any of claim 1, wherein the implant has a length less than 30% of a length of a maximal extent of a bone for which said implant is designed.

3. An implant according to claim 1, 3rd alternative, wherein said shaft has at least one end adapted to attach an object thereto.

4. An implant according to claim 1, 3rd alternative, wherein said shaft has two ends each adapted to attach an object thereto.

5. An implant according to any of claims 1-4, comprising a base portion (206, 328) having a greater diameter than said shaft and adapted to remain outside said bone.

6. An implant according to claim 5, wherein said base is adapted to urge an object against an outside of said bone.

7. An implant according to claim 6, wherein said object comprises a bone plate.

8. An implant, according to claim 5, wherein said base is adapted for attachment of an object thereto.

9. An implant according to claim 8, wherein said object comprises a rod.

10. An implant according to claims 9, wherein said rod has a thin surface texture.

11. An implant according to claim 9, wherein said surface texture comprises a plurality of radial notches.

12. An implant according to any of claims 1-2, comprising a base portion having a diameter that is not greater than said shaft and adapted to remain inside said bone.

13. An implant according to any of claims 1-2, comprising a separate axial over tube (1152) adapted to be fixed to said bone and sized to enclose said shaft.

14. An implant according to claim 13, wherein said shaft is free to move axially in said tube.

15. An implant according to claim 13, wherein said shaft is rigidly coupled to said tube.

16. An implant according to any of claims 15, comprising a second shaft having at least a second inflatable anchor portion defined thereon and comprising a coupling between said shaft and said second shaft.

17. An implant according to claim 16, wherein said second shaft is adapted for axial inserting in said bone.

18. An implant according to claim 16 or claim 17, wherein said coupling comprises a through hole in said second shaft.

19. An implant according to claim 16 or claim 17, wherein said coupling comprises a through hole in said shaft.

20. An implant according to claim 16 or claim 17, wherein said coupling comprises a notch in said second shaft.

21. An implant according to any of claims 16-20, comprising a strut adapted to couple to said first and second shafts.

22. An implant according to any of claims 1-20, comprising a strut adapted to at least contact said shaft.

23. An implant according to any of claims 1-22, wherein said inflatable anchor portions is adapted to expand wholly in cancellus bone and not contact cortical bone.

24. An implant according to any of claims 1-23, comprising an expanding cortical anchoring portion, adapted to expand in contact with cortical bone.

25. An implant according to any of claims 1-24, comprising a second inflatable cancellus anchoring portion, adapted to engage a cancellus bone portion of another section of said bone, separated by a fracture line from a section which anchors said anchoring portion.

26. An implant according to any of claims 1-25, wherein said shaft is not inflatable.

27. An implant according to any of claims 1-26, wherein at least part of said shaft is externally threaded.

28. An implant according to claim 27, wherein said threading is adapted to engage cortical bone.

29. An implant according to claim 27, wherein said threading is adapted to engage cancellus bone.

30. An implant according to any of claims 1-29, wherein at least part of said anchoring portion is externally threaded.

31. An implant according to claim 30, wherein said anchoring portion threading is damaged by said inflation.

32. An implant according to claim 30, wherein said anchoring portion threading is not damaged by said inflation.

33. An implant according to any of claims 1-32, comprising matched threading on said anchoring portion and said shaft.

34. An implant according to any of claims 1-32, wherein said inflation plastically deforms said inflatable portion.

35. An implant according to any of claims 1-34, wherein said shaft and said inflatable portion are comprised of a metal.

36. An implant according to any of claims 35, wherein at least a part of said inflatable portion is metallurgically treated to have better elongation properties than said shaft.

37. An implant according to any of claims 1-36, wherein said shaft defines a guide channel for a guiding element.

38. An implant according to any of claims 37, wherein said guide channel comprises a lumen.

39. An implant according to any of claims 37, wherein said guide channel comprises a slot along an exterior part of said shaft.

40. An implants according to any of claims 1-39, wherein said plurality of protrusions comprises at least one axially aligned bar.

41. An implant according to any of claims 1-40, wherein said plurality of protrusions comprises at least one trans-axially aligned bar.

42. An implant according to any of claims 1-41, wherein said plurality of protrusions comprises a spirally arranged bar.

43. An implant according to any of claims 1-42, wherein said membrane has a smooth surface texture.

44. An implant according to any of claims 1-42, wherein said membrane has a rough surface texture.

45. An implant according to any of claims 1-44, wherein said inflatable portion and said shaft have substantially a same diameter prior to inflation.

46. An implant according to any of claims 1-44, wherein said inflatable portion has a substantially smaller diameter than said shaft prior to inflation.

47. An implant according to any of claims 1-44, wherein said inflatable portion has a substantially greater diameter than said shaft prior to inflation.

48. An implant according to any of claims 1-47, wherein said implant has a length of less than 150 mm.

49. An implant according to any of claims 1-48, wherein said implant has a shaft diameter of less than 15 mm.

50. An implant according to any of claims 1-49, wherein said implant has an expansion ratio of at least 1:1.2 of its diameter before and after inflation.

51. An implant according to any of claims 1-49, wherein said implant has an expansion ratio of at least 1:1.4 of its diameter before and after inflation.

52. An implant according to any of claims 1-51, wherein said implant is adapted to withstand an inflation pressure of at least 103.42 kPa (15 PSI).

53. An implant according to any of claims 1-51, wherein said implant is adapted to withstand an inflation pressure of at least 689.48 kPa (100 PSI).

54. An implant according to any of claims 1-53, wherein said implant is provided in a sterile packaging including an indication of a use of said implant.

55. An implant according to claim 1, wherein said implant is adapted for use as a pedicle anchoring screw.

56. An implant according to claim 1, wherein said implant is adapted for use as a compression hip nail.

57. An implant according to claim 1, wherein said implant is adapted for use as a dynamic hip nail.

58. An implant according to claim 1, wherein said implant is adapted for joining fractures of condyles.

## Patentansprüche

1. Ein Implantat (200, 300), das zum Verankern in Spongiosa angepasst ist, aufweisend:
einen Schaft (208, 308), der eine Längsachse hat und dadurch hindurch ein Aufblaslumen (338) definiert, und
mindestens einen aufblasbaren Ankerabschnitt, der eine dünne aufblasbare flexible Membran aufweist und mit dem Lumen verbunden ist, wobei ein Aufblasen des Ankerabschnitts bewirkt, dass das Implantat mit der Spongiosa in Eingriff gelangt, wobei der Ankerabschnitt eine Mehrzahl von Vorsprüngen (222, 1158) aufweist, die sich von der Membran aus senkrecht oder in einem Winkel zu einer Fläche der Membran erstrecken und angepasst sind, um mit Spongiosa in Eingriff zu sein, wobei sich der Ankerabschnitt an einer distalen oder proximalen Spitze (216, 316) des Schaftes befindet, oder sich an einem zentralen Abschnitt des Schaftes befindet, und wobei der Ankerabschnitt weniger als 60% einer Länge des Implantats aufweist.

2. Ein Implantat gemäß Anspruch 1, wobei das Implantat eine Länge von weniger als 30% einer Länge einer maximalen Erstreckung eines Knochens hat, für den das Implantat bestimmt ist.

3. Ein Implantat gemäß Anspruch 1, dritte Alternative, wobei der Schaft mindestens ein Ende hat, das angepasst ist, um ein Objekt daran anzubringen.

4. Ein Implantat gemäß Anspruch 1, dritte Alternative, wobei der Schaft zwei Enden hat, die jeweils angepasst sind, um ein Objekt daran anzubringen.

5. Ein Implantat gemäß irgendeinem der Ansprüche 1 bis 4, aufweisend einen Basisabschnitt (206, 328), der einen größeren Durchmesser als der Schaft hat und angepasst ist, um außerhalb des Knochens zu verbleiben.

6. Ein Implantat gemäß Anspruch 5, wobei die Basis angepasst ist, um ein Objekt gegen eine Außenseite des Knochens zu drängen.

7. Ein Implantat gemäß Anspruch 6, wobei das Objekt eine Knochenplatte aufweist.

8. Ein Implantat gemäß Anspruch 5, wobei die Basis für ein Anbringen eines Objektes daran angepasst ist.

9. Ein Implantat gemäß Anspruch 8, wobei das Objekt eine Stange aufweist.

10. Ein Implantat gemäß Anspruch 9, wobei die Stange eine dünne Oberflächenbeschaffenheit hat.

11. Ein Implantat gemäß Anspruch 9, wobei die Oberflächenbeschaffenheit eine Mehrzahl von radialen Nuten aufweist.

12. Ein Implantat gemäß irgendeinem der Ansprüche 1 bis 2, aufweisend einen Basisabschnitt, der einen Durchmesser hat, der nicht größer als der Schaft ist und angepasst ist, um innerhalb des Knochens zu verbleiben.

13. Ein Implantat gemäß irgendeinem der Ansprüche 1 bis 2, aufweisend ein getrenntes axiales Überrohr (1152), das angepasst ist, um an dem Knochen befestigt zu sein, und bemessen ist, um den Schaft zu umschließen.

14. Ein Implantat gemäß Anspruch 13, wobei sich der Schaft in dem Rohr frei axial bewegen kann.

15. Ein Implantat gemäß Anspruch 13, wobei der Schaft steif mit dem Rohr verbunden ist.

16. Ein Implantat gemäß irgendeinem der Ansprüche 1 bis 15, aufweisend einen zweiten Schaft, der mindestens einen zweiten Ankerabschnitt aufweist, der daran definiert ist, und aufweisend eine Verbindung zwischen dem Schaft und dem zweiten Schaft.

17. Ein Implantat gemäß Anspruch 16, wobei der zweite Schaft zum axialen Einsetzen in den Knochen angepasst ist.

18. Ein Implantat gemäß Anspruch 16 oder Anspruch 17, wobei die Verbindung in dem zweiten Schaft ein Durchgangsloch aufweist.

19. Ein Implantat gemäß Anspruch 16 oder Anspruch 17, wobei die Verbindung in dem Schaft ein Durchgangsloch aufweist.

20. Ein Implantat gemäß Anspruch 16 oder Anspruch 17, wobei die Verbindung in dem zweiten Schaft eine Nut aufweist.

21. Ein Implantat gemäß irgendeinem der Ansprüche 16 bis 20, aufweisend eine Strebe, die angepasst ist, um den ersten und den zweiten Schaft miteinander zu verbinden.

22. Ein Implantat gemäß irgendeinem der Ansprüche 1 bis 20, aufweisend eine Strebe, die angepasst ist, um den Schaft mindestens zu kontaktieren.

23. Ein Implantat gemäß irgendeinem der Ansprüche 1 bis 22, wobei der aufblasbare Ankerabschnitt angepasst ist, um sich in Spongiosa vollständig zu entfalten und die Kortikalis nicht zu kontaktieren.

24. Ein Implantat gemäß irgendeinem der Ansprüche 1 bis 23, aufweisend einen expandierenden Kortikalis-Verankerungsabschnitt, der angepasst ist, um in Kontakt mit Kortikalis zu expandieren.

25. Ein Implantat gemäß irgendeinem der Ansprüche 1 bis 24, aufweisend einen zweiten aufblasbaren Spongiosa-Verankerungsabschnitt, der angepasst ist, um mit einem Spongiosa-Abschnitt eines anderen Abschnitts des Knochens in Eingriff zu sein, der durch eine Bruchlinie von einem Abschnitt getrennt ist, der den Verankerungsabschnitt verankert.

26. Ein Implantat gemäß irgendeinem der Ansprüche 1 bis 25, wobei der Schaft nicht aufblasbar ist.

27. Ein Implantat gemäß irgendeinem der Ansprüche 1 bis 26, wobei zumindest ein Teil des Schaftes ein Außengewinde aufweist.

28. Ein Implantat gemäß Anspruch 27, wobei das Gewinde angepasst ist, um mit der Kortikalis in Eingriff zu sein.

29. Ein Implantat gemäß Anspruch 27, wobei das Gewinde angepasst ist, um mit Spongiosa in Eingriff zu sein.

30. Ein Implantat gemäß irgendeinem der Ansprüche 1 bis 29, wobei zumindest ein Teil des Verankerungsabschnitts ein Außengewinde aufweist.

31. Ein Implantat gemäß Anspruch 30, wobei das Verankerungsabschnittsgewinde durch das Aufblasen beschädigt wird.

32. Ein Implantat gemäß Anspruch 30, wobei das Verankerungsabschnittsgewinde durch das Aufblasen nicht beschädigt wird.

33. Ein Implantat gemäß irgendeinem der Ansprüche 1 bis 32, aufweisend ein zusammenpassendes Gewinde an dem Verankerungsabschnitt und dem Schaft.

34. Ein Implantat gemäß irgendeinem der Ansprüche 1 bis 32, wobei das Aufblasen den aufblasbaren Abschnitt plastisch verformt.

35. Ein Implantat gemäß irgendeinem der Ansprüche 1 bis 34, wobei der Schaft und der aufblasbare Abschnitt aus einem Metall zusammengesetzt sind.

36. Ein Implantat gemäß Anspruch 35, wobei mindestens ein Teil des aufblasbaren Abschnitts metallurgisch behandelt ist, um bessere Dehnungseigenschaften als der Schaft zu haben.

37. Ein Implantat gemäß irgendeinem der Ansprüche 1 bis 36, wobei der Schaft einen Führungskanal für ein Führungselement definiert.

38. Ein Implantat gemäß Anspruch 37, wobei der Führungskanal ein Lumen aufweist.

39. Ein Implantat gemäß Anspruch 37, wobei der Führungskanal entlang einem äußeren Teil des Schaftes einen Schlitz aufweist.

40. Ein Implantat gemäß irgendeinem der Ansprüche 1 bis 39, wobei die Mehrzahl von Vorsprüngen mindestens eine axial ausgerichtete Stange aufweist.

41. Ein Implantat gemäß irgendeinem der Ansprüche 1 bis 40, wobei die Mehrzahl von Vorsprüngen mindestens eine transaxial ausgerichtete Stange aufweist.

42. Ein Implantat gemäß irgendeinem der Ansprüche 1 bis 41, wobei die Mehrzahl von Vorsprüngen eine wendelförmig angeordnete Stange aufweist.

43. Ein Implantat gemäß irgendeinem der Ansprüche 1 bis 42, wobei die Membran eine glatte Oberflächenbeschaffenheit hat.

44. Ein Implantat gemäß irgendeinem der Ansprüche 1 bis 42, wobei die Membran eine raue Oberflächenbeschaffenheit hat.

45. Ein Implantat gemäß irgendeinem der Ansprüche 1 bis 44, wobei der aufblasbare Abschnitt und der Schaft vor dem Aufblasen im Wesentlichen einen gleichen Durchmesser haben.

46. Ein Implantat gemäß irgendeinem der Ansprüche 1 bis 44, wobei der aufblasbare Abschnitt vor dem Aufblasen einen im Wesentlichen kleineren Durchmesser als der Schaft hat.

47. Ein Implantat gemäß irgendeinem der Ansprüche 1 bis 44, wobei der aufblasbare Abschnitt vor dem Aufblasen einen im Wesentlichen größeren Durchmesser als der Schaft hat.

48. Ein Implantat gemäß irgendeinem der Ansprüche 1 bis 47, wobei das Implantat eine Länge von weniger als 150 mm hat.

49. Ein Implantat gemäß irgendeinem der Ansprüche 1 bis 48, wobei das Implantat einen Schaftdurchmesser von weniger als 15 mm hat.

50. Ein Implantat gemäß irgendeinem der Ansprüche 1 bis 49, wobei das Implantat ein Expansionsverhältnis von mindestens 1:1,2 seines Durchmessers vor und nach dem Aufblasen hat.

51. Ein Implantat gemäß irgendeinem der Ansprüche 1 bis 49, wobei das Implantat ein Expansionsverhältnis von mindestens 1:1,4 seines Durchmessers vor und nach dem Aufblasen hat.

52. Ein Implantat gemäß irgendeinem der Ansprüche 1 bis 51, wobei das Implantat angepasst ist, um einem Aufblasdruck von mindestens 103,42 kPa (15 PSI) standzuhalten.

53. Ein Implantat gemäß irgendeinem der Ansprüche 1 bis 51, wobei das Implantat angepasst ist, um einem Aufblasdruck von mindestens 689,48 kPa (100 PSI) standzuhalten.

54. Ein Implantat gemäß irgendeinem der Ansprüche 1 bis 53, wobei das Implantat in einer sterilen Verpackung vorgesehen ist, die eine Angabe einer Verwendung des Implantats enthält.

55. Ein Implantat gemäß Anspruch 1, wobei das Implantat zur Verwendung als eine Bogenfuß-Verankerungsschraube angepasst ist.

56. Ein Implantat gemäß Anspruch 1, wobei das Implantat zur Verwendung als ein Kompressionshüftnagel angepasst ist.

57. Ein Implantat gemäß Anspruch 1, wobei das Implantat zur Verwendung als ein dynamischer Hüftnagel angepasst ist.

58. Ein Implantat gemäß Anspruch 1, wobei das Implantat zum Zusammenfügen von Gelenkkopffrakturen angepasst ist.

## Revendications

1. Implant (200, 300) adapté pour s'ancrer dans un os spongieux, comprenant :
une gaine (208, 308) ayant un axe longitudinal et définissant une lumière de gonflage (338) à travers elle ; et
au moins une partie d'ancrage gonflable comprenant une fine membrane souple gonflable et raccordée à ladite lumière, moyennant quoi le gonflage de ladite partie d'ancrage amène ledit implant à se mettre en prise avec ledit os spongieux, dans lequel ladite partie d'ancrage comprend une pluralité de saillies (222, 1158) s'étendant à partir de ladite membrane perpendiculairement à ou selon un angle par rapport à une surface de la membrane et adaptées pour mettre en prise l'os spongieux, dans lequel ladite partie d'ancrage est au niveau d'une pointe distale ou proximale (216, 316) de ladite gaine ou est au niveau d'une partie centrale de ladite gaine, et dans lequel ladite partie d'ancrage comprend moins de 60 % d'une longueur dudit implant.

2. Implant selon la revendication 1, lequel implant a une longueur inférieure à 30 % d'une longueur d'une étendue maximale d'un os pour lequel ledit implant est conçu.

3. Implant selon la revendication 1, troisième variante, dans lequel ladite gaine a au moins une extrémité adaptée pour y attacher un objet.

4. Implant selon la revendication 1, troisième variante, dans lequel ladite gaine a deux extrémités, chacune adaptée pour y attacher un objet.

5. Implant selon l'une quelconque des revendications 1 à 4, comprenant une partie de base (206, 328) ayant un plus grand diamètre que ladite gaine et adaptée pour rester à l'extérieur dudit os.

6. Implant selon la revendication 5, dans lequel ladite base est adaptée pour pousser un objet contre un extérieur dudit os.

7. Implant selon la revendication 6, dans lequel ledit objet comprend une plaque osseuse.

8. Implant selon la revendication 5, dans lequel ladite base est adaptée pour y attacher un objet.

9. Implant selon la revendication 8, dans lequel ledit objet comprend une tige.

10. Implant selon la revendication 9, dans lequel ladite tige a une texture de surface fine.

11. Implant selon la revendication 9, dans lequel ladite texture de surface comprend une pluralité d'encoches radiales.

12. Implant selon l'une quelconque des revendications 1 à 2, comprenant une partie de base ayant un diamètre qui n'est pas supérieur à ladite gaine et adaptée pour rester à l'intérieur dudit os.

13. Implant selon l'une quelconque des revendications 1 à 2, comprenant un surtube axial séparé (1152) adapté pour être fixé sur ledit os et dimensionné pour enfermer ladite gaine.

14. Implant selon la revendication 13, dans lequel ladite gaine est libre de se déplacer axialement dans ledit tube.

15. Implant selon la revendication 13, dans lequel ladite gaine est rigidement couplée audit tube.

16. Implant selon l'une quelconque des revendications 1 à 15, comprenant une seconde gaine ayant au moins une seconde partie d'ancrage gonflable définie sur elle et comprenant un couplage entre ladite gaine et ladite seconde gaine.

17. Implant selon la revendication 16, dans lequel ladite seconde gaine est adaptée pour une insertion axiale dans ledit os.

18. Implant selon la revendication 16 ou la revendication 17, dans lequel ledit couplage comprend un trou traversant dans ladite seconde gaine.

19. Implant selon la revendication 16 ou la revendication 17, dans lequel ledit couplage comprend un trou traversant dans ladite gaine.

20. Implant selon la revendication 16 ou la revendication 17, dans lequel ledit couplage comprend une encoche dans ladite seconde gaine.

21. Implant selon l'une quelconque des revendications 16 à 20, comprenant une entretoise adaptée pour se coupler auxdites première et seconde gaines.

22. Implant selon l'une quelconque des revendications 1 à 20, comprenant une entretoise adaptée pour au moins être en contact avec ladite gaine.

23. Implant selon l'une quelconque des revendications 1 à 22, dans lequel lesdites parties d'ancrage gonflables sont adaptées pour s'étendre complètement dans l'os spongieux et ne pas être en contact avec l'os cortical.

24. Implant selon l'une quelconque des revendications 1 à 23, comprenant une partie d'ancrage d'os cortical s'étendant, adaptée pour s'étendre en contact avec l'os cortical.

25. Implant selon l'une quelconque des revendications 1 à 24, comprenant une seconde partie d'ancrage spongieuse gonflable, adaptée pour mettre en prise une partie d'os spongieux d'une autre section dudit os, séparée, par une ligne de rupture, d'une section qui ancre ladite partie d'ancrage.

26. Implant selon l'une quelconque des revendications 1 à 25, dans lequel ladite gaine n'est pas gonflable.

27. Implant selon l'une quelconque des revendications 1 à 26, dans lequel au moins une partie de ladite gaine est filetée extérieurement.

28. Implant selon la revendication 27, dans lequel ledit filetage est adapté pour mettre en prise l'os cortical.

29. Implant selon la revendication 27, dans lequel ledit filetage est adapté pour mettre en prise l'os spongieux.

30. Implant selon l'une quelconque des revendications 1 à 29, dans lequel au moins une partie de ladite partie d'ancrage est filetée extérieurement.

31. Implant selon la revendication 30, dans lequel ledit filetage de partie d'ancrage est endommagé par ledit gonflage.

32. Implant selon la revendication 30, dans lequel ledit filetage de partie d'ancrage n'est pas endommagé par ledit gonflage.

33. Implant selon l'une quelconque des revendications 1 à 32, comprenant un filetage correspondant sur ladite partie d'ancrage et ladite gaine.

34. Implant selon l'une quelconque des revendications 1 à 32, dans lequel ledit gonflage déforme plastiquement ladite partie gonflable.

35. Implant selon l'une quelconque des revendications 1 à 34, dans lequel ladite gaine et ladite partie gonflable sont composées d'un métal.

36. Implant selon la revendication 35, dans lequel au moins une partie de ladite partie gonflable est traitée métallurgiquement pour avoir de meilleures propriétés d'élongation que ladite gaine.

37. Implant selon l'une quelconque des revendications 1 à 36, dans lequel ladite gaine définit un canal de guidage pour un élément de guidage.

38. Implant selon la revendication 37, dans lequel ledit canal de guidage comprend une lumière.

39. Implant selon la revendication 37, dans lequel ledit canal de guidage comprend une fente le long d'une section extérieure de ladite gaine.

40. Implant selon l'une quelconque des revendications 1 à 39, dans lequel ladite pluralité de saillies comprend au moins une barre alignée de manière axiale.

41. Implant selon l'une quelconque des revendications 1 à 40, dans lequel ladite pluralité de saillies comprend au moins une barre alignée de manière transaxiale.

42. Implant selon l'une quelconque des revendications 1 à 41, dans lequel ladite pluralité de saillies comprend une barre agencée en spirale.

43. Implant selon l'une quelconque des revendications 1 à 42, dans lequel ladite membrane a une texture de surface lisse.

44. Implant selon l'une quelconque des revendications 1 à 42, dans lequel ladite membrane a une texture de surface rugueuse.

45. Implant selon l'une quelconque des revendications 1 à 44, dans lequel ladite partie gonflable et ladite gaine ont sensiblement le même diamètre avant le gonflage.

46. Implant selon l'une quelconque des revendications 1 à 44, dans lequel ladite partie gonflable a un diamètre sensiblement plus petit que ladite gaine avant le gonflage.

47. Implant selon l'une quelconque des revendications 1 à 44, dans lequel ladite partie gonflable a un diamètre sensiblement supérieur à ladite gaine avant le gonflage.

48. Implant selon l'une quelconque des revendications 1 à 47, dans lequel ledit implant a une longueur inférieure à 150 mm.

49. Implant selon l'une quelconque des revendications 1 à 48, dans lequel ledit implant a un diamètre de gaine inférieur à 15 mm.

50. Implant selon l'une quelconque des revendications 1 à 49, dans lequel ledit implant a un rapport d'expansion d'au moins 1:1,2 de son diamètre avant et après gonflage.

51. Implant selon l'une quelconque des revendications 1 à 49, dans lequel ledit implant a un rapport d'expansion d'au moins 1:1,4 de son diamètre avant et après gonflage.

52. Implant selon l'une quelconque des revendications 1 à 51, dans lequel ledit implant est adapté pour résister à une pression de gonflage d'au moins 103,42 kPa (15 PSI).

53. Implant selon l'une quelconque des revendications 1 à 51, dans lequel ledit implant est adapté pour résister à une pression de gonflage d'au moins 689,48 kPa (100 PSI).

54. Implant selon l'une quelconque des revendications 1 à 53, dans lequel ledit implant est prévu dans un emballage stérile comprenant une indication d'utilisation dudit implant.

55. Implant selon la revendication 1, dans lequel ledit implant est adapté pour être utilisé en tant que vis d'ancrage pédiculaire.

56. Implant selon la revendication 1, dans lequel ledit implant est adapté pour être utilisé en tant que clou de compression de hanche.

57. Implant selon la revendication 1, dans lequel ledit implant est adapté pour être utilisé en tant que clou dynamique de hanche.

58. Implant selon la revendication 1, dans lequel ledit implant est adapté pour assembler les fractures des condyles.
